(19) Europäisches Patentamt
European Patent Office
Office européen des brevets

(11) Veröffentlichungsnummer: **0 474 323 A1**

(12) # EUROPÄISCHE PATENTANMELDUNG

(21) Anmeldenummer: **91250221.8**

(22) Anmeldetag: **06.08.91**

(51) Int. Cl.[5]: **C07C 217/84**, C07C 217/20, C07F 13/00, C07D 495/04, C07C 331/28, C07K 15/28, A61K 49/02

(30) Priorität: **10.08.90 DE 4025788**

(43) Veröffentlichungstag der Anmeldung: **11.03.92 Patentblatt 92/11**

(84) Benannte Vertragsstaaten: **AT BE CH DE DK ES FR GB GR IT LI LU NL SE**

(71) Anmelder: **INSTITUT FÜR DIAGNOSTIKFORSCHUNG GmbH, c/o FU KLINIKUM RUDOLF VIRCHOW**
**Standort Charlottenburg Haus 9, Spandauer Damm 130**
**W-1000 Berlin 19(DE)**

(72) Erfinder: **Hahn, Ekkehardt Dr.**
**Reichssportfeldstr. 16**
**W-1000 Berlin 19(DE)**
Erfinder: **Rupprecht, Stefan Dipl.-Chem.**
**Teichstr. 67**
**W-1000 Berlin 51(DE)**
Erfinder: **Kramp,Wolfgang Dr.**
**Damwildsteig 41 A**
**W-1000 Berlin 27(DE)**
Erfinder: **Neumeier, Reinhard Dr.**
**Gabrielenstr. 63**
**W-1000 Berlin 27(DE)**

(54) **Reduzierende Chelatbildner, deren Technetium- und Rhenium-Komplexe, Verfahren zu ihrer Herstellung sowie deren Verwendung in Diagnostik und Therapie.**

(57) Verbindungen der allgemeinen Formel I

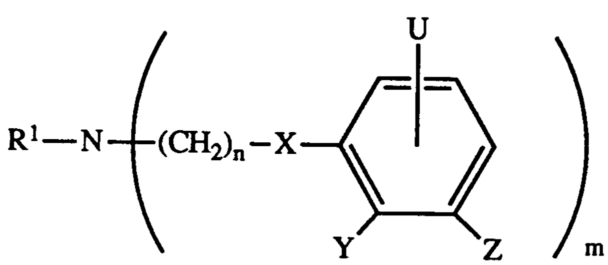

(I)

haben die Fähigkeit Pertechnetat ohne Zusatz von Reduktionsmitteln zu einer Oxidationsstufe Kleiner als +7 zu reduzieren, über die Gruppen Y und Z stabile Komplexe mit den so reduzierten Technetium oder Rheniumisotopen zu bilden und sich gegebenenfalls aufgrund einer - mit Hilfe einer in $R^1$ enthaltenen funktionellen Gruppe - gekoppelten Verbindung selektiv in bestimmten Geweben oder Läsionen anzureichern. Die Technetium- und Rhenium-Komplexe der Verbindungen der allgemeinen Formel I, sowie deren Salze mit organischen und anorganischen Säuren lassen sich in der In-vivo-Diagnostik und Therapie, bevorzugt der Tumortherapie, einsetzen.

EP 0 474 323 A1

Seit längerer Zeit werden radioaktive Metallionen, meist gebunden an einen Komplexbildner, für die In-vivo-Diagnostik verwendet. Hiervon ist Technetium-99m (Tc-99m) aufgrund seiner für diese Zwecke nahezu idealen physikalischen Eigenschaften - gute Absorption der Strahlung in entsprechenden Detektionsgeräten (Gammakamera, SPECT-Geräte) gegenüber einer geringen Absorption im menschlichen Organismus und leichten Verfügbarkeit über einen Molybdän/Technetium-Generator - das am häufigsten in der klinischen Nuklearmedizin verwendete Radionuklid. Seine kurze Halbwertszeit von 6,02 h garantiert eine nur geringe Belastung des Patienten mit Gammastrahlung, zumal auch das Tochternuklid Technetium-99 nur eine vernachlässigbare Reststrahlung besitzt Ein Nachteil des Technetiums ist allerdings seine komplizierte und noch nicht vollständig bekannte Komplex-Chemie. Technetium kann in einer Reihe von Oxidationsstufen (+7 bis -1) vorliegen, wobei sich die pharmakologischen Eigenschaften durch Veränderung der Ladung eines Komplexes stark verändern können. Es ist deswegen nötig, Komplexe zu verwenden, die das Technetium in einer definierten Oxidationstufe binden und Redox-Reaktionen, die zu einer Redistribution des Pharmakons führen könnten, zu verhindern.

Für eine organ- oder gewebespezifische Diagnostik ist es notwendig, daß sich die Radiopharmaka selektiv in den gewünschten Zielorganen bzw. -geweben anreichern und dort eine Zeitlang verbleiben. Diese Selektivität kann einmal durch die Entwicklung von Komplexen erreicht werden, die von sich aus eine Spezifität für bestimmte Gewebe zeigen, oder durch Kopplung der Technetium-Komplexe an selektive Substanzen wie z.B. monoklonale Antikörper.

Zur Markierung organspezifischer Substanzen mit Tc-99m muß das aus dem Nuklidgenerator eluierte Pertechnetat zuerst in eine niedrigere Oxidationsstufe überführt werden. In dieser reduzierten Form bildet Technetium mit den sich selektiv anreichernden Substanzen mehr oder weniger stabile Verbindungen. Das besondere Problem der Markierung mit Tc-99m besteht darin, daß normalerweise Zinn(-II-)Ionen in der Reaktionslösung als Reduktionsmittel vorhanden sind. Zinn-II ist bisher das einzige Reduktionsmittel, das eine schnelle und quantitative Überführung des Pertechnetats bei Raumtemperatur in eine niedrigere und dadurch reaktionsfähige Oxidationsstufe ermöglicht. Dabei müssen die zugesetzten Zinn-II-salze in einem hohen Überschuß (ca. 100 : 1) gegenüber Pertechnetat eingesetzt werden. Die nach erfolgter Reduktion neben dem reduzierten Tc-99m vorliegenden Zinn-II und -IV-Ionen konkurrieren jedoch um die Bindungsstellen des Liganden, so daß entweder der Komplexbildner wiederum im Überschuß gegenüber dem Zinn eingesetzt werden muß, wodurch die spezifischen Aktivität stark herabgesetzt wird, oder ungebundenes Tc-99m und Zinn als gemeinsames Colloid zu unerwünschter Radioaktivitätsspeicherung in anderen Organen führt. In beiden Fällen wird die diagnostische Aussagekraft gemindert.

Dieses Problem kann durch die Verwendung reduzierender Liganden umgangen werden. Bei der Herstellung von Diagnostika nach diesem Prinzip wirkt ein Teil des zugesetzten Ligandenüberschusses als Reduktionsmittel für Pertechnetat, das Technetium in eine Oxidationsstufe kleiner als +7 reduziert. Auf diese Weise reduzierte Technetium-Spezies werden dann vom Überschuß des nicht oxidierten Chelatbildners komplexiert. Dabei ist es wichtig, stabile Komplexe in einer definierten Oxidationsstufe für das Technetium zu erhalten.

DeLearie et al. (L. A. deLearie, R. C. Haltiwanger, C. G. Pierpont; J. Am. Chem. Soc. 111: 4324, 1989) zeigten, daß 3,5-Di-tert-butylcatechole zur Reduktion und Chelatbildung von Tc-99 (Halbwertzeit: 212.000 Jahre) geeignet sind. Die Reduktion erfolgte durch 24 Stunden Kochen in Methanol. Für die Markierung mit dem kurzlebigen Isotop des Technetiums (Tc-99m; Halbwertzeit: 6 Stunden) sind jedoch nur Substanzen als Radiopharmaka anwendbar, die schnell und schonend auch in der Klinik markierbar sind und für die keine anschließende Aufreinigung nach der Markierung mit z.B. Tc-99m erforderlich ist. Die von DeLearie beschriebenen Verbindungen sind somit für die Herstellung eines klinisch anwendbaren Radiopharmakons ungeeignet.

Gegenstand der vorliegenden Erfindung ist es, neue reduzierende und gewebsspezifische Chelatbildner, sowie deren stabile Technetium- und Rhenium-Komplexe zur Verfügung zu stellen. Überraschenderweise konnten Substanzen gefunden werden, die sowohl unter schonenden Bedingungen als auch schnell Tc-99m reduzieren und vollständig komplexieren. Sie lassen sich zudem im Gegensatz zu den vorher beschriebenen Verbindungen auch zur Kopplung an sich selektiv in Krankheitsherden oder bestimmten Geweben anreichernde Substanzen verwenden.

Erfindungsgemäß wird diese Aufgabe durch Verbindungen der allegemeinen Formel I gelöst

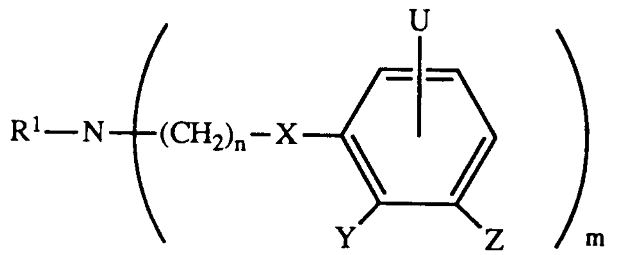

(I)

worin X für -O-, -S-, $-NR^2-$ mit $R^2$ in der Bedeutung eines Wasserstoffatoms oder eines $C_{1-6}$-Alkylrestes, Y und Z gleich oder verschieden sind und für die Reste -OH, $-NHR^3$ oder $-SR^3$ mit $R^3$ in der Bedeutung eines Wasserstoffatoms oder eines $C_{1-6}$-Alkylrestes und
U für ein Wasserstoffatom, einen verzweigten oder unverzweigten $C_{1-6}$-Alkyl-, einen $C_{1-6}$-Alkoxy-, einen Hydroxyl- oder einen Carboxylrest stehen,
n die Ziffern 2 bis 6 und
m die Ziffern 2 oder 3 bedeutet und $R^1$ nur vorhanden ist, wenn m für die Ziffer 2 steht,
$R^1$ für ein Wasserstoffatom, einen Benzyl-, oder einen verzweigten oder unverzweigten gegebenenfalls mit ein bis drei Hydroxyl-, Carboxyl- oder Aminogruppen substituierten $C_{0-6}$-Alkylrest steht, der gegebenenfalls eine funktionelle Gruppe B oder eine - gegebenenfalls mit Hilfe dieser funktionellen Gruppe gebundene - sich selektiv in Läsionen oder bestimmten Geweben anreichernde Verbindung T enthält,

wobei B im Falle, daß Y und/oder Z für $NHR^3$ steht, für einen Amino-, einen Hydrazino- oder Hydrazid-, einen Carboxyl-, einen $C_{1-6}$-Alkinyl- oder -Alkenyl-, einen Hydroxyl-, einen Aminophenyl-, einen Oxiranyl-, einen fluorierten Phenoxycarbonyl- oder einen Biotinrest,

oder im Falle, daß Y und Z für -OH oder $SR^3$ steht, zusätzlich auch für einen Halogen-, einen Formyl-, einen Nitril-, einen Phenylisothiocyanato- oder einen gegebenenfalls mit einem Natriumsulfatrest substituierten Succinimidoxycarbonylrest,

und T für monoklonale Antikörper oder deren Fragmente, Hormone, Wachstumsfaktoren, Liganden für Zellmembranrezeptoren, Steroide, Neurotransmitter, Fettsäuren, Sacharide, Aminosäuren und Oligopeptide, Biotin, sowie Radiosensitizer wie z.B. Misonidazol steht,
wobei in $R^1$ gegebenenfalls vorhandene funktionelle Gruppen gegebenenfalls in geschützter Form oder deren Vorstufen vorliegen,
mit Ausnahme der Verbindung $N\{CH_2\text{-}CH_2\text{-}CH_2\text{-}O\text{-}C_6H_3\text{-}2,3\text{-}(OH)_2\}_3$,
deren Technetium- und Rhenium-Komplexe, sowie deren Salze mit anorganischen und organischen Säuren.

Die ausgenommene Verbindung $N\{CH_2\text{-}CH_2\text{-}CH_2\text{-}O\text{-}C_6H_3\text{-}2,3\text{-}(OH)_2\}_3$ ist bekannt (B. Wolff, Angew. Chem. 98:173, 1986) und wurde zur Komplexierung von Germanium und Silizium verwendet.

Als Säuren werden zur Salzbildung physiologisch verträgliche anorganische Säuren wie z.B. Salz- oder Schwefelsäure und organische Säuren wie z.B. Essig- oder Citronensäure verwendet.

Unter einem $C_0$-Alkylrest ist eine direkte Bindung zu verstehen.

Erfindungsgemäß bevorzugt sind diejenigen Verbindungen, in deren allgemeiner Formel I U ein Wasserstoffatom und X ein Sauerstoffatom darstellen, n die Ziffer 3 ist, Y und Z gleich sind und für OH- oder $NH_2$- stehen und $R^1$ ein Wasserstoffatom, einen Benzylrest, einen unverzweigten gegebenenfalls mit einer Hydroxyl- oder Aminogruppe substituierten $C_{0-3}$-Alkylrest darstellt, der gegebenenfalls eine funktionelle Gruppe B oder - mit Hilfe von B gekoppelt - monoklonale Antikörper bzw. deren Fragmente, Steroide oder Misonidazol enthält.

Die erfindungsgemäßen Substanzen besitzen überraschenderweise die Vorzüge, daß sie

1. Pertechnetat aus einem Nuklidgenerator unter milden Bedingungen, schnell und ohne Zusatz von Reduktionsmitteln zu einer Oxidationsstufe kleiner als +7 reduzieren können,
2. stabile Komplexe mit dem so reduzierten Technetium ohne weiteren Zusatz von Reduktionsmitteln bei neutralem pH-Wert bilden,
3. sich aufgrund der gegebenenfalls mit Hilfe der funktionellen Gruppe B gekoppelten Verbindungen T, wie monoklonale Antikörper oder deren Fragmente, Hormone, Wachstumsfaktoren, Liganden für Zellmembranrezeptoren, Steroide, Neurotransmitter, Fettsäuren, Sacharide, Aminosäuren und Oligopeptide, Biotin sowie Radiosensitizer wie z.B. Misonidazol selektiv in bestimmten Geweben oder Läsionen anreichern

bzw.

ohne eine Gruppe T zu enthalten eine Anreicherung in bestimmten Geweben oder Läsionen zeigen.

Die Bildung der Technetiumkomplexe der oben beschriebenen Chelate erfolgt mit $TcO_4^-$ aus einem Nuklidgenerator bei neutralem pH-Wert ohne Zusatz von Reduktionsmittel in wäßriger Lösung.

Diese Eigenschaft der hier beschriebenen Chelatbildner bietet wesentliche Vorteile gegenüber bisher bekannten Liganden. Der Einbau von Zinn, welches bei den bekannten Ligandensystemen als Reduktionsmittel zugesetzt werden muß, in das Chelat wird vermieden. Die Bildung von Tc-99m Spezies, die nicht durch die oben beschriebenen Chelate gebunden werden (Beispiele 6 und 7), wurde nicht beobachtet. Die erfindungsgemäßen Chelate sind damit eindeutig besser für diagnostische Zwecke geeignet als die bisher bekannten Chelate.

Ihre Herstellung erfolgt dadurch, daß man Amine der allgemeinen Formel II,

$R^{1'}$—N{—$(CH_2)_n$—Nu}$_m$ (II)

worin Nu für ein Nucleofug wie z.B. Cl, Br, I, $CH_3C_6H_4SO_3$, $CH_3SO_3$ oder $CF_3SO_3$

und $R^{1'}$ für einen Substituenten $R^1$, dessen gegebenenfalls vorhandenen funktionellen Gruppen in geschützter Form oder als deren Vorstufen vorliegen und der keine sich selektiv anreichernde Verbindung T enthält, stehen

mit Aromaten der allgemeinen Formel III

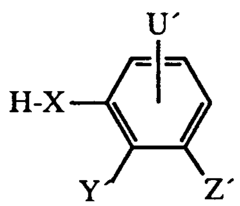

(III)

worin U' für einen Substituenten U, dessen Hydroxy- oder Carboxylrest in geschützter Form vorliegt, und Y' und Z' für Y und Z oder deren Vorstufen oder in geschützter Form stehen,

unter Basenkatalyse in polaren Lösungsmitteln bei Temperaturen von 50 - 200 °C innerhalb von 6 Stunden bis 6 Tagen, vorzugsweise 2 Stunden bis 4 Tage, umsetzt

und anschließend die in $R^1$ gegebenenfalls enthaltene funktionelle Gruppe B oder die gewünschten Aromat-Substituenten Y und Z generiert, gewünschtenfalls die so erhaltenen kopplungs- bzw. komplexierungsfähigen Verbindungen mit derjeweils gewünschten sich selektiv anreichernden Verbindung T koppelt bzw. mit dem jeweils gewünschten Technetium- oder Rhenium-Isotop komplexiert - wobei die Reihenfolge der Schritte Kopplung an T und Komplexierung mit dem Technetium- oder Rhenium-Isotop vertauscht werden kann - und anschließend die noch vorhandenen Schutzgruppen entfernt bzw. die Vorstufen in die letztendlich gewüschten Substituenten überführt.

Als Hydroxyschutzgruppen kommen z.B. die Benzyl-, 4-Methoxybenzyl-, 4-Nitrobenzyl-, Trityl-, Diphenylmethyl-, Trimethylsilyl-, Dimethyl-t-butylsilyl- und Diphenyl-t-butylsilyl-gruppe infrage. Im Falle von Polyolen können die Hydroxygruppen auch in Form von Ketalen mit z.B. Aceton, Acetaldehyd, Cyclohexanon oder Benzaldehyd geschützt sein. Ferner können die Hydroxygruppen auch z.B. als THP-Ether, α-Alkoxyethylether, MEM-Ether oder als Ester mit aromatischen oder aliphatischen Carbonsäuren, wie z.B. Essigsäure oder Benzoesäure, vorliegen.

Die Hydroxyschutzgruppen können nach den dem Fachmann bekannten Literaturmethoden, z.B. durch Hydrogenolyse, reduktive Spaltung mit Lithium / Ammoniak, Säurebehandlung der Ether und Ketale oder Alkalibehandlung der Ester freigesetzt werden (siehe z.B. "Protective Groups in Organic Synthesis", T.W. Greene, John Wiley and Sons 1981).

Als Säureschutzgruppen kommen niedere Alkyl-, Aryl- und Aralkylgrüppen, beispielsweise die Methyl-, Ethyl-, Propyl-, n-Butyl-, t-Butyl-, Phenyl-, Benzyl-, Diphenylmethyl-, Triphenylmethyl, bis(p-Nitrophenyl)-methylgruppe, sowie Trialkylsilylgruppen infrage.

Die Abspaltung der Schutzgruppen erfolgt nach dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50 °C, saure Verseifung mit Mineralsäuren oder im Fall von z.B. tert.-Butylestern mit Hilfe von Trifluoressigsäure.

Für die Herstellung von Verbindungen der allgemeinen Formel I mit Y und Z in der Bedeutung von SH-Gruppen geht man aus von Ligandenvorstufen der allgemeinen Formel III mit Y'und z' in der Bedeutung

$SR^3$-Resten. Die Abspaltung der Schutzgruppen nach der Umsetzung mit den Aminen der allgemeinen Formel II erfolgt wahlweise mit Alkalialkylthiolaten, Alkalialkoholaten oder Alkalimetallen, vorzugsweise mit Natriummethylthiolat in einem polaren Lösungsmittel, vorzugsweise in HMPT, DMF oder Dimethylacetamid.

Als Aminoschutzgruppen kommen z.B. Trifluoracetyl-, t-Butoxycarbonyl-, 2,2,2-Trichloroethoxycarbonyl-, Benzoxycarbonyl- und Acetylgruppen infrage. Die Aminoschutzgruppen können nach literaturbekannten Methoden, z.B. durch basische oder saure Hydrolyse, reduktive Spaltung mit Zink in Essigsäure oder Hydrogenolyse abgespalten werden.

Enthält die Ligandenvorstufe der Formel III funktionalisierte Aromaten mit Y' und Z' in der Bedeutung von $NO_2$-Gruppen, dann erfolgt die Herstellung des Chelatbildners gemäß Anspruch 1 durch Reduktion, vorzugsweise mit Zinn in salzsaurer Lösung.

Bei Verbindungen gemäß der allgemeinen Formel I, in denen m die Ziffer 2 bedeutet, läßt sich der Rest $R^{1'}$ modifizieren. Ist $R^{1'}$ zum Beispiel eine Benzylgruppe, so kann diese durch Umsetzung mit Wasserstoff unter erhöhtem Druck und erhöhter Temperatur in der Gegenwart eines Palladiumkatalysators entfernt werden. Der Rest $R^1$ ist dann ein Wasserstoffatom.

Rhenium- 186 besitzt eine physikalische Halbwertzeit von 3,7 Tagen und emittiert Betapartikel mit einer für die Therapie von z.B. Tumoren geeigneten Energie von 1,1 MeV sowie gleichzeitig Gammastrahlung mit einer Energie von 137 keV (9% Häufigkeit). Rhenium befindet sich im Periodensystem in Gruppe VII A direkt unter Technetium und weist praktisch eine identische Struktur- und Chelat-Chemie wie Technetium auf. Diese Eigenschaften machen Rhenium-186 zu einem idealen Isotop für therapeutische Anwendungen (Schädigung des erkrankten Gewebes durch partikuläre Betastrahlung) mit der gleichzeitigen Möglichkeit zur diagnostischen Überprüfung einer Anreicherung mit Hilfe des Anteils an Gammastrahlung. Rhenium-188, das ebenfalls zur Tumortherapie verwendet werden kann, hat eine wesentlich kürzere Halbwertzeit von 17 Stunden und eine Betaenergie von 2,1 MeV. Auch Rhenium-188 hat einen Gammastrahlen-Anteil (155 keV; 15%) und kann somit auch zur Therapie und gleichzeitigen Detektion mit einer Gammakamera herangezogen werden.

Wenn die mit einem radioaktiven Isotop komplexierten Chelatbildner von sich aus keine Selektivität für Läsionen oder bestimmte Gewebe zeigen, ist es notwendig, daß sie an eine selektive Substanz gekoppelt werden. Der Rest $R^1$ ist z.B. mit Hilfe der funktionellen Gruppe B dazu geeignet, eine stabile Verbindung zu Proteinen oder anderen sich selektiv anreichernden Molekülen herzustellen. Durch die entsprechende Wahl der funkionellen Gruppe gelingt die Kopplung unter schonenden Reaktionsbedingungen, die die biologische Funktion und/oder Selektivität nicht beeinflussen.

Die Kopplung an die gewünschten Verbindungen erfolgt ebenfalls nach an sich bekannten Methoden, (z.B. Fritzberg et al.; J. Nucl. Med.: 26, 7 [1987]), beispielsweise durch Reaktion der Gruppe B mit nucleophilen Gruppen des sich selektiv anreichernden Moleküls oder, wenn es sich bei der Gruppe B selbst um ein Nucleophil handelt, mit aktivierten Gruppen des sich selektiv anreichernden Moleküls.

Die Gruppe B repräsentiert jeden Substituenten, der einerseits eine funktionelle Gruppe darstellt, die eine Kopplung an ein sich selektiv anreicherndes Molekül unter milden Bedingungen erlaubt (z.B. durch Acylierung oder Amidierung) sowie jede aktivierte Gruppe, die mit nucleophilen Gruppen von Proteinen, Antikörpern, Hormonen oder anderen Biomolekülen reagieren kann, wie der Amino-, Phenol-, Sulfhydryl-, Aldehyd- oder Imidazol-Gruppe. Unter aktivierter Gruppe wird eine Funktion verstanden, die fähig ist, unter Bildung eines Konjugates mit einem nucleophilen Substituenten eines selektiven Moleküls oder des Komplexliganden selbst in wässriger Lösung innerhalb einer angemessen kurzen Zeit, unter Reaktionsbedingungen, die weder Denaturierung noch Verlust der biologischen Aktivität bzw. Selektivität zufolge haben, zu reagieren. Beispiele dafür sind Imidester, Alkylimidester, Amidoalkylimidester, Succinimidester, Acylsuccinimide, Phenolester, substituierte Phenolester, Tetrafluorphenolester, Anhydride, Hydrazide, Alkylhalogenide und Michael-Akzeptoren. B ist bevorzugt ein Monoanhydrid, Säurechlorid, Säurehydrazid, gemischtes Anhydrid, aktivierter Ester (wie Phenol- oder Imid-Ester), Nitren oder Isothiocyanat, insbesondere für die Kopplung mit nucleophilen Gruppen von Aminosäuren oder ein aliphatisches oder aromatisches primäres Amin für die Kopplung an Kohlenhydratreste von Proteinen.

Handelt es sich bei der Gruppe B selbst um ein Nucleophil, kann sie mit aktivierten Gruppen eines sich selektiv anreichernden Moleküls reagieren, wobei auch mit sogenannten "Cross-linking-reagents" umgesetzte Gruppen des selektiven Moleküls mit eingeschlossen sind. Als "Cross-Linker" können homobifunktionelle Imidoester, homobifunktionelle N-Hydroxysuccinimid-Ester (NHS) sowie heterobifunktionelle "Cross-Linker", die unterschiedliche funktionelle Gruppen, wie NHS-Ester, Pyridyl-Disulfide und aktivierte Halogene, wie $\alpha$-Keto-Halogenide enthalten, eingesetzt werden. Solche Cross-Linker sind kommerziell erhältlich.

Als Kopplungspartner werden sich selektiv in bestimmten Geweben oder Läsionen anreichernde Verbindungen verwendet. Vielfach konnte die selektive Anreicherung dieser Substanzen bereits durch Markierung mit Positronen-emittierenden Isotopen (PET-Technik), Iod-Isotopen oder anderen Kopplungspartnern gezeigt werden. Z.T. kamen bereits auch mit Tc-99m markierte Verbindungen zur Verwendung. Solchermaßen Technetium-markierte Verbindungen haben jedoch den Nachteil, daß Zinn-II-Ionen als Reduktionsmittel hinzugefügt werden müssen, was zu den oben beschriebenen Konsequenzen (erniedrigte spezifische Radioaktivität und die Möglichkeit, daß ungebundenes Tc-99m zusammen mit Zinn als Colloid zu einer unerwünschten Radioaktivitätsspeicherung in anderen Organen und einer verminderten diagnostischen Aussagekraft) führt.

Liganden, die an spezifische Rezeptoren binden, können ein in ihrer Rezeptordichte verändertes Gewebe erkennen; hierzu gehören u.a. Peptid- und Steroid-Hormone, Wachstumsfaktoren und Neurotransmitter. Mit Liganden für Steroidhormon-Rezeptoren wurde die Möglichkeit einer verbesserten Diagnostik von Brust- und Prostatacarcinomen aufgezeigt (S.J. Brandes & J.A. Katzenellenbogen, Nucl. Med. Biol. 15: 53, 1988). Vielfach konnten mit Positronenemittierenden Isotopen markierte Liganden für Neurorezeptoren zur Diagnostik verschiedener Hirnerkrankungen herangezogen werden (J.J. Forst, Trends in Pharmacol. Sci. 7: 490,1987). Verschiedentlich weisen Tumorzellen eine veränderte Dichte von Rezeptoren für Peptidhormone oder Wachstumsfaktoren, wie z.B. den "epidermal growth factor" (EGF) auf. Die Konzentrationsunterschiede konnten zur selektiven Anreicherung von Cytostatika in Tumorzellen genutzt werden (E. Aboud-Pirak et al., Proc. Natl. Acad. Sci. USA 86: 3778, 1989).

Weitere Biomoleküle sind in den Metabolismus der Zellen einschleusbare Metaboliten, die einen veränderten Stoffwechsel erkennbar machen; hierzu gehören beispielsweise Lipide (auch in Form von Liposomen), Saccharide, Porphyrine, Peptide und Aminosäuren. Fettsäuren gekoppelt mit Tc-Chelatbildner wurden in der EPA 0 200 492 beschrieben. Andere Stoffwechselprodukte wie Saccharide (Dexoxyglucose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med. 8, 10, 1986). Bestimmte Porphyrine zeigten eine Anreicherung in Tumoren (P.A. Scourides, Cancer Res. 47: 3439, 1987).

Auch nicht-biologische Substanzen wie Misonidazol und seine Derivate, die sich in Geweben bzw. Gewebeteilen mit reduzierter Sauerstoffkonzentration irreversibel an Zellbestandteile binden, können zur spezifischen Anreicherung von radioaktiven Isotopen und somit bildlichen Darstellung von Tumoren oder ischämischen Regionen herangezogen werden (M.E. Shelton, J. Nucl. Med. 30: 351, 1989). Andere nicht-biologische Substanzen sind sich in Tumoren anreichernde Cytostatika wie Bleomycin. Auch geeignete Polymere wie Dextrane, Polyethylenimine, Polyamide, Polyharnstoffe, Polyether und Polythioharnstoffe kommen als Kopplungspartner in Betracht.

Die Biotin enthaltenden erfindungsgemäßen Verbindungen ermöglichen die Bindung der radioaktiven Konjugate an Avidin- oder Streptavidin-haltige Substanzen. Dies kann genutzt werden, um Antikörper-Streptavidin-Konjugate am Tumor anzureichern und die radioaktive Biotin-haltige Komponente erst später zu applizieren, was zu einer verringerten Exposition des Patienten mit radioaktiver Strahlung führt (D.J. Hnatowich et al., J. Nucl. Med. 28: 1294, 1987). Schließlich ist auch die direkte Kopplung der bifunktionellen Chelatbildner an Proteine wie z.B. monoklonale Antikörper bzw. deren Fragmente, Albumin, Enzyme (z.B. Urokinase, Streptokinase), Fibrin, Fibrinogen oder Myosin möglich.

Durch Komplexierung der Konjugate mit Tc-99m bzw. Rhenium-Isotopen wird eine Diagnostik und Therapie von Tumoren oder anderer Erkrankungen ermöglicht. Hierbei ist es unerheblich, ob eine Markierung der Chelatbildner mit Tc-99m oder einem Rhenium-Isotop vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, sowie keine anschließenden Aufreinigung erforderlich ist.

Die Herstellung der erfindungsgemäßen pharmazeutischen Mittel erfolgt in an sich bekannter Weise, in dem man die erfindungsgemäßen Komplexbildner - gegebenenfalls unter Zugabe der in der Galenik üblichen Zusätze - in wäßrigem Medium löst und anschließend sterilfiltriert. Geeignete Zusätze sind beispielsweise physiologisch unbedenkliche Puffer (z.B. Tromethamin), geringe Zusätze von Elektrolyten (z.B. Natriumchlorid), Stabilisatoren (z.B. Gluconat oder Phosphonate) und geringe Mengen von Oxidations- oder Reduktionsmitteln (10-500 $\mu$g/-Dosis). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Bei der nuklearmedizinischen In-vivo-Anwendung werden die erfindungsgemäßen Mittel in Mengen von $1 \cdot 10^{-5}$ bis $5 \cdot 10^{4}$ nmol/kg Körpergewicht, vorzugsweise in Mengen zwischen $1 \cdot 10^{-3}$ und $5 \cdot 10^{2}$ nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 - 350 mCi

appliziert. Die Applikation wird normalerweise durch intravenöse, intraarterielle, peritoneale oder intratumorale Injektion von 0,1 bis 2 ml einer Lösung der erfindungsgemäßen Mittel erfolgen. Bevorzugt ist die intravenöse Applikation.

Die nachfolgenden Beispiele dienen der näheren Erläuterung des Erfindungsgegenstandes.

**Beispiel 1:**

***Tri(β-carbethoxyethyl)amin, 1 und Di(β-carbethoxyethyl)amin, 2***

In einem Bombenrohr werden 300 ml frisch destillierter Acrylsäureethylester mit 300 ml flüssigem Ammoniak einen Tag lang zur Reaktion gebracht und das entstandene Produktgemisch wird, nach Entfernung letzter Ammoniakreste auf dem Wasserbad, im Vakuum fraktioniert destilliert. Die erste Fraktion bildet Bis-(β-carbethoxy-ethyl)amin, **2**, (Kp. 97-110 ° C/0.05 mbar), die Hauptfraktion besteht aus dem gewünschten Produkt, **1**, (Kp. 120-133 ° C/0.05 mbar).
Ausbeute: 144 g (49%) Tri(β-carbethoxyethyl)amin, **1**
$^{1}$H-NMR ($CDCl_3$, δ, ppm):
4,10 (q, 2H, $C(O)OC\mathit{H_2}CH_3$); 2,74 (t, 2H, $NC\mathit{H_2}CH_2$); 2,41 (t, 2H, $CH_2C\mathit{H_2}C(O)O$); 1,23 (t, 3H, $OCH_2C\mathit{H_3}$)
$^{13}$C-NMR ($CDCl_3$, δ, ppm):
172,2 ($CH_2\mathit{C}(O)O$); 60,1 ($C(O)O\mathit{C}H_2CH_3$); 49,1 ($N\mathit{C}H_2CH_3$); 32,8 ($CH_2\mathit{C}H_2C(O)O$); 14,1 ($OCH_2\mathit{C}H_3$)
Für Di(β-carbethoxyethyl)amin **2**, wird gefunden:
$^{1}$H-NMR ($CDCl_3$, δ, ppm):
4,12 (q, 4H, $C(O)OC\mathit{H_2}CH_3$); 2,88 (t, 4H, $NC\mathit{H_2}CH_2$); 2,47 (t, 4H, $CH_2C\mathit{H_2}C(O)O$); 1,60 (s, br, 1H, $\mathit{H}N(CH_2)_2$); 1,24 (t, 6H, $OCH_2C\mathit{H_3}$)

***Tris(3-hydroxypropyl)amin, 3***

In einem 2l-Dreihalskolben mit Tropftrichter und Rückflußkühler werden 18 g (0,7 mol) Lithiumaluminiumhydrid in 900 ml absolutem Ether suspendiert. Dazu wird innerhalb einer Stunde eine Lösung von 77 g (0,24 mol) des Esters 1 in 200 ml absolutem Ether so zugetropft, daß die Lösung mäßig siedet. Nach fünfstündigem Rühren bei 25 ° C und vorsichtiger Hydrolyse überschüssigen Hydrids mit Wasser trennt man das Produkt über einen Büchnertrichter von ausgefallenen Hydroxiden ab. Nach Entfernen des Lösungsmittels im Vakuum wird der Rückstand kurz in Ethanol aufgekocht, weiteres $LiAl(OH)_4$ über eine Fritte (D3) abgenutscht, der Alkohol abgezogen und die verbleibende Flüssigkeit in Methylenchlorid aufgenommen. Druckloses Filtern durch eine weitere Fritte (D4) und Abziehen des Lösungsmittels im Vakuum ergibt eine honiggelbe, hochviskose Flüssigkeit.
Ausbeute: 27,3 g (59 %)
$^{1}$H-NMR ($CDCl_3$, δ, ppm):
3,78 (t, 2H, $CH_2C\mathit{H_2}OH$); 2,62 (t, 2H, $NC\mathit{H_2}CH_2$); 1,80 (q, 2H, $CH_2C\mathit{H_2}CH_2$);
$^{13}$C-NMR ($CDCl_3$, δ, ppm):
60,6 ($CH_2\mathit{C}H_2OH$); 51,3 ($N\mathit{C}H_2CH_2$); 28,1 ($CH_2\mathit{C}H_2CH_2$)

***Tris(3-chlorpropyl)amin,* 4**

Zu 8,6 g (45 mmol) Tris(3-hydroxypropyl)amin, **3**, gelöst in 80 ml Chloroform werden 18,9 g (160 mmol) Thionylchlorid gegeben. Dabei entsteht eine unlösliche weiße Masse, die sich langsam wieder auflöst. Nachdem die Reaktionsmischung drei Stunden unter Rückfluß gekocht hat, wird nach Abkühlung überschüssiges $SOCl_2$ mit Wasser hydrolysiert. Die organische Phase wird viermal mit 50 ml heißem Wasser ausgeschüttelt, die vereinigten wässrigen Phasen mit 40 %iger Natronlauge stark alkalisch gemacht und danach viermal mit je 80 ml Ether extrahiert. Nach Trocknen über $Na_2SO_4$ und Entfernen des Ethers in Vakuum wird der gelbliche Rückstand fraktioniert destilliert, wobei das Produkt als farblose Flüssigkeit übergeht und nach längerem Stehen bei Raumtemperatur auskristallisiert. Tris(3-chlorpropyl)amin läßt sich aus Ethanol umkristallisieren (3 g **4** auf 7 ml Ethanol).
Ausbeute 9,6 g (87 %)
Fp.: 35 ° C
Kp.: 120 ° C /0.05 mbar
$^{1}$H-NMR ($CDCl_3$, δ, ppm):
3,60 (t, 2H, $CH_2C\mathit{H_2}Cl$); 2,52 (t, 2H, $NC\mathit{H_2}CH_2$); 1,88 ppm (q, 2H, $CH_2C\mathit{H_2}CH_2$)
$^{13}$C-NMR ($CDCl_3$, δ, ppm):

50,5 ($CH_2CH_2Cl$); 43,0 ($NCH_2CH_2$); 30,1 ($CH_2CH_2CH_2$)

***2,2-Dimethyl-1,3-benzodioxol-4-ol,* 5**

In einem 500 ml Zweihalskolben mit Tropftrichter und einer Kolonne nach Widmer werden 77 g (0,61 mol) Pyrogallol in 250 ml absolutem Toluol suspendiert und erhitzt. Wenn das Lösungsmittel zu Sieden beginnt, werden 75 ml (0,61 mol) 2,2-Dimethoxypropan zugegeben. Am Kolonnenkopf geht daraufhin stetig Destillat bei etwa 60 ° C über. Nach zwei Stunden werden weitere 75 ml Dimethoxypropan zugegeben. Nach Absinken der Temperatur am Kolonnenkopf (ca. sechs Stunden) wird die Reaktionsmischung zur Vervollständigung der Umsetzung über Nacht unter Rückfluß gekocht. Die abgekühlte Lösung wird im Vakuum vom Toluol befreit und der zähe Rückstand über eine Brücke mit großem Querschnitt destilliert. Hierbei kristallisiert das Produkt an der Brückenwand schon aus und muß durch Erhitzen in die Vorlage überführt werden. Die Destillationstemperatur wird so gewählt, daß vorhandene gelbliche Verunreinigungen nur begrenzt überdestillieren. Das leicht gelbliche Produkt läßt sich bei 75 ° C, 0,2 mbar sublimieren. Das Acetal ist gut löslich in Aceton und Methanol, schwer löslich in Chloroform.

Variante zur Aufarbeitung: Nach Entfernen des Toluols wird das Rohprodukt in soviel heißen Tetrachlorkohlenstoff aufgenommen, daß es sich gerade löst. Beim Abkühlen auf Raumtemperatur kristallisiert das weiße Produkt **5** auf dem Lösungsmittel aus. Es wird nach gleichen Bedingungen wie beschrieben sublimiert. Ein signifikanter Unterschied in den Ausbeuten ist nicht festzustellen.

Ausbeute: 50 g (50 %)
Fp.: 90 ° C
$^1$H-NMR ($CDCl_3$, $\delta$, ppm):
6,68 (t, 1H, Ar-*H*); 6,46 (d, 1H, Ar-*H*); 6,40 (d, 1H, Ar-*H*); 5,13 (s, 1H, Ar-O*H*); 1,69 (s, 6H, $CH_3$)
$^{13}$C-NMR (MeOD, $\delta$, ppm):
149,7, 141,8, 135,3, 122,1 (Ar); 118,6 ($C(CH_3)_2$); 111,3 (Ar); 101,6 (Ar); 25,8 ($C(CH_3)_2$)

***Tris[3-(2,2-dimethyl-1,3-benzodioxol-4-yloxy)propyl]amin,* 6**

In einem ausgeflammten 250 ml Zweihalsschlenkkolben mit Rückflußkühler werden 20,1 g (121 mmol) getrocknetes Pyrogallolacetal **5** vorgelegt und in 100 ml absolutem (99 %) Ethanol gelöst. Zum Entfernen letzter Sauerstoffreste wird der Kolben alternierend fünfmal entgast und mit Argon belüftet. Dann werden 4,75 g (121 mmol) Kaliummetall in kleinen Stücken zugegeben. Aus der Lösung fällt zunächst weißes Kaliumphenolat aus, das sich alsbald wieder auflöst. Nun wird zu der jetzt überaus sauerstoffempfindlichen Lösung eine ebenfalls entgaste Lösung aus 9,6 g (39 mmol) Tri(3-chlorpropyl)amin und 20 ml Ethanol zugespritzt. Nachdem die Lösung vier Tage unter Rückfluß gekocht hat, werden 4 ml Eisessig zugegeben, ausgefallenes KCl von der noch heißen aber nicht mehr luftempfindlichen Lösung abfiltriert und der Filterkuchen mit wenig heißem Ethanol nachgewaschen. Durch Stehenlassen bei Raumtemperatur kristallisiert die Ligandenvorstufe langsam aus. Die Kristalle werden abfiltriert und mit eiskaltem Ethanol nachgewaschen. Eine zweite Fraktion kann durch Einengen der Mutterlauge gewonnen werden.

Ausbeute: 11,5g (46 %)
Fp.: 65 ° C
$^1$H-NMR ($CDCl_3$, $\delta$, ppm):
6,66 (t, 1H, Ar-*H*); 6,42 (d, 1H, Ar-*H*); 6,26 (d, 1H, Ar-*H*); 3,95 (t, 2H, $CH_2CH_2O$); 2,59 (t, 2H, $NCH_2CH_2$); 1,91 (q, 2H, $CH_2CH_2CH_2$); 1,68 (s, 6H, $C(CH_3)_2$)
$^{13}$C-NMR ($CDCl_3$, $\delta$, ppm):
148,3 (Ar); 143,2 (Ar); 135,2 (Ar); 121, (Ar); 117,9 ($C(CH_3)_2$); 107,9 (Ar); 102,0 (Ar); 66,8 ($CH_2CH_2O$); 49,8 ($NCH_2CH_2$); 26,9 ($CH_2CH_2CH_2$); 25,7 ($C(CH_3)_2$)

***Tris-(3-(2,3-dihydroxyphenoxy)propyl)aminhydrochlorid,* 7**

39,6 g (62 mmol) **6** werden unter Argon in 250 ml Eisessig gelöst und zum Sieden erhitzt. Dazu werden 200 ml eines Gemisch aus 50 % Eisessig, 20 % Wasser und 30 % rauchender Salzsäure innerhalb von zwei Stunden zugetropft. Das Lösungsmittel wird abdestilliert, so daß etwa 200ml im Kolben verbleiben. Die Lösung wird langsam abgekühlt. Die ausgefallenen gelblichen Kristalle werden abfiltriert und in wenig heißem Eisessig umkristallisiert. Das so erhalterne weiße Pulver wird bei 90 ° C, $10^{-3}$ rnbar zwei Tage am Ölpumpenvakuum getrocknet.

Ausbeute: 31,7g (92 %)
Fp.: 190 ° C

$^1$H-NMR ([$D_6$]-DMSO, δ, ppm):
10,47 (s, br, 1/3H, N*H*); 8,93 (s, br, 1H, O*H*); 8,19 (s, br, 1H, O*H*); 6,42-6,54 (m, 3H, Ar-*H*); 4,02 (m, br, nicht aufgelöst, 2H, $CH_2C\mathit{H_2}O$); 3,40 (m, br, nicht aufgelöst, 2H, $NC\mathit{H_2}CH_2$); 2,17 (m, br, nicht aufgelöst, 2H, $CH_2C\mathit{H_2}CH_2$)
$^{13}$C-NMR ([$D_6$]-DMSO, δ, ppm):
147,5, 146,2, 134,7, 118,5, 109,6, 105,2 (Ar); 66,2 ($CH_2\mathit{C}H_2O$); 49,9 ($N\mathit{C}H_2CH_2$); 23,4 ($CH_2\mathit{C}H_2CH_2$)

**Beispiel 2:**

***Benzyl-di(β-carbethoxyethyl)amin,* 8**

172g (1,60 mol) Benzylamin werden in 500 ml Ethanol vorgelegt und unter Eiskühlung mit 384 g (3,84 mol) Acrylsäureester versetzt. Die Reaktionsmischung wird 5 Tage bei Raumtemperatur gerührt. Lösungsmittel und überschüssige Edukte werden am Rotationsverdampfer abgezogen. Die zurückbleibende Lösung wird im Vakuum fraktioniert destilliert.

| | |
|---|---|
| Fraktion 1: | < 140 Grad/0,05 mbar |
| Fraktion 2: | 140 - 145 Grad/0,05 mbar |
| Fraktion 3: | 145 - 148 Grad/0,05 mbar |
| Fraktion 4: | 145 - 150 Grad/0,05 mbar |

Ausbeute: 380 g (77 %) **8** aus Fraktion 4
$^1$H-NMR ($CDCl_3$, δ, ppm):
7,27 (m, 5H, Ar-*H*); 4,10 (q, 4H, $C(O)OC\mathit{H_2}CH_3$); 3,59 (s, 2H, $C_6H_5C\mathit{H_2}N$); 2,80 (t, 4H, $NC\mathit{H_2}CH_2$); 2,46 (t, 4H, $CH_2C\mathit{H_2}C(O)$); 1,23 (t, 6H, $OCH_2C\mathit{H_3}$)
$^{13}$C-NMR ($CD_3OD$, δ, ppm):
173,7 ($CH_2\mathit{C}(O)O$); 140,2, 129,7, 129,0, 127,9 (Ar); 61,1 ($O\mathit{C}H_2CH_3$); 59,1 ($C_6H_5\mathit{C}H_2N$); 51,1 ($N\mathit{C}H_2CH_2$); 33,5 ($CH_2\mathit{C}H_2C(O)$); 14,5 ($OCH_2\mathit{C}H_3$)

***Benzyl-bis(3-hydroxypropyl)amin,* 9**

19g (0,5 mol) Lithiumaluminiumhydrid wird in 900 ml Ether vorgelegt und unter Eiskühlung werden 92 g (0,3 mol) des Esters **8** langsam zugetropft. Die Lösung wird 12 h bei Raumtemperatur gerührt und dann vorsichtig mit Wasser hydrolysiert. Der Ether und die wässrige Phase werden vom ausgefallenen $LiAl(OH)_4$ abdekantiert. Der Feststoff wird mehrmals mit Ether gewaschen. Die vereinigten organischen Phasen (Ether und Ethanol) werden im Scheidetrichter vom Wasser abgetrennt, über $MgSO_4$ getrocknet und filtriert. Nach Entfernen des Lösungsmittels im Vakuum bleibt das Produkt als farblose Flüssigkeit zurück.
Ausbeute: 60,5 g (90 %)
$^1$H-NMR ($CDCl_3$, δ, ppm):
7,30 (m, 5H, Ar-*H*); 4,11 (s, br, 2H, $CH_2O\mathit{H}$); 3,67 (t, 4H, $CH_2C\mathit{H_2}OH$); 3,56 (s, 2H, $C_6H_5C\mathit{H_2}N$); 2,61 (t, 4H, $NC\mathit{H_2}CH_2$); 1,75 (q, 4H, $CH_2C\mathit{H_2}CH_2$)
$^{13}$C-NMR ($CD_3OD$, δ, ppm):
137,8, 130,1, 129,2, 128,0 (Ar); 61,9 ($CH_2\mathit{C}H_2OH$); 59,5 ($C_6H_5\mathit{C}H_2N$; 52,3 ($N\mathit{C}H_2CH_2$); 30,2 ($CH_2\mathit{C}H_2CH_2$)

***Benzyl-bis(3-chlorpropyl)amin,* 10**

143g (0,64 mol) **9** werden in 600 ml Chloroform vorgelegt. Dazu tropft man bei Raumtemperatur 182 g (1,53 mol) Thionylchlorid, gelöst in 100 ml Chloroform. Die Zugabe muß so erfolgen, daß das Lösungsmittel mäßig siedet. Nach beendeter Zugabe wird 3 h unter Rückfluß gekocht. Die abgekühlte Lösung wird vorsichtig mit Wasser hydrolysiert und zweimal mit 300 ml heißem Wasser gewaschen. Nun wird die organische Phase stark eingeengt und weitere zweimal mit je 250 ml heißem Wasser ausgeschüttelt. Nach Vereinigung der wässrigen Phasen werden diese mit Natronlauge (40 %) stark alkalisch gemacht und zweimal mit je 400 ml Ether extrahiert. Die vereinigten Etherextrakte werden über Natriumsulfat getrocknet. Danach wird das Lösungsmittel am Rotationsverdampfer abgezogen. Das Rohprodukt wird fraktioniert destilliert.
Ausbeute: 140 g (84 %)
Sdp.: 114-125°C
$^1$H-NMR ($CDCl_3$, δ, ppm):
7,30 (m, 5H, Ar-*H*); 3,58 (t, 4H, $CH_2C\mathit{H_2}Cl$); 3,55 (s, 2H, $C_6H_5C\mathit{H_2}N$); 2,58 (t, 4H, $NC\mathit{H_2}CH_2$); 1,92 (q, 4H, $CH_2C\mathit{H_2}CH_2$)

$^{13}$C-NMR ($CD_3OD$, δ, ppm):
140,3, 129,9, 129,2, 128,0 (Ar); 59,8 ($C_6H_5$*C*$H_2$N); 51,9 (N*C*$H_2CH_2$); 43,8 ($CH_2$*C*$H_2$Cl); 31,3 ($CH_2$*C*$H_2CH_2$)

***Benzyl-bis[(3-(2,2-dimethyl-1,3-benzodioxol-4-yloxy]propyl)amin,*** **11**

In einem ausgeflammten 250 ml Zweihalsschlenkkolben werden 13.3 g (80 mmol) des Ketals **5** eine halbe Stunde im Hochvakuum bei 50 Grad getrocknet. Danach wird 5 in 100 ml absolutem (99 %) Ethanol gelöst. Die Lösung wird mehrmal bis zum Aufsieden evakuiert und mit Argon belüftet. Blank geschnittenes Kalium (3,1 g, 80 mmol) wird zugegeben und zu der jetzt sauerstoffempfindlichen Lösung wird das sauerstofffreie Chlorid **10** (9,7 g, 37,3 mmol) zugegeben. Der Ansatz wird 3 Tage unter Rückfluß gekocht. Zur Aufarbeitung werden 3 ml Eisessig zugegeben und das ausgefallene Kaliumchlorid noch heiß abfiltriert. Das Filtrat wird trocken gezogen und in einem Gemisch von ca. 30 ml Pentan/Ether 1:1 gelöst. Die braune Lösung wird auf einer kurzen Säule (etwa 50 g Silicagel) mit Ether/Pentan 1:1 eluiert. Dabei ist darauf zu achten, daß dunkel gefärbte Produkte nicht miteluiert werden. Nach dem Abziehen des Laufmittels bleibt ein gelbes Öl zurück, das aus Ethanol umkristallisiert wird (16 g in 200 ml Ethanol). Hierbei fällt das Produkt bei Raumtemperatur als farblose Kristallen an.
Ausbeute: 16 g (80 %)
Fp.: 46-47 °C
$^1$H-NMR ([$D_6$]-Aceton, δ, ppm):
7.30 (dd, 2H, Benzylaromat); 7,20 (m, 3H, Benzylaromat); 6,67 (dd, 2H, Brenzkatechin); 6,41 (dd, 4H, Brenzkatechin); 4,07 (t, 4H, $CH_2$*C*$H_2$O); 3,58 (s, 2H, $C_6H_5$*CH*$_2$N); 2,61 (t, 4H, N*CH*$_2CH_2$); 1,91 (q, 4H, $CH_2$*CH*$_2CH_2$); 1,60 (s, 12H, C(*CH*$_3$)$_2$)
$^{13}$C-NMR ($CD_3OD$, δ, ppm):
149,3, 144,1 (Brenzkatechin); 140,7 (Benzylaromat); 136,1 (Brenzkatechin); 129.4, 128.8, 127,4 (Benzylaromat); 121,9 (Brenzkatechin); 118,4 (*C*($CH_3$)$_2$); 109,4, 102,6 (Brenzkatechin); 67,8 ($CH_2$*C*$H_2$O); 59,3 ($C_6H_5$*C*$H_2$N); 50,7 (N*C*$H_2CH_2$); 27,9 ($CH_2$*C*$H_2CH_2$); 25,8 (C(*C*$H_3$)$_2$)

***Benzyl-bis[(3-(2,3-dihydroxyphenoxy)propyl]aminhydrochlorid,*** **12**

14 g (27 mmol) der Ligandenvorstufe **11** werden in 100 ml Eisessig gelöst und zum Sieden erhitzt. Dazu gibt man innerhalb von zwei Stunden 100 ml eines Säuregemisches (50 % Eisessig, 20 % Wasser, 30 % rauchende Salzsäure), wobei der durch abdestillierendes Lösungsmittel entstehende Flüssigkeitsverlust durch freiwerdendes Aceton ausgeglichen wird. Nach beendeter Zugabe wird soviel Lösungsmittel abdestilliert, daß etwa 50 ml im Kolben verbleiben. Die heiße Lösung wird langsam abgekühlt. Das Produkt kann aber so nicht ausgefällt werden. Zieht man alle Lösungsmittel ab, fällt der Ligand als voluminöser Rückstand an. Dieses Rohprodukt wird durch Waschen mit Ether von letzten Essigsäureresten befreit und in Hochvakuum getrocknet.
Ausbeute: 12,4 g (96 %)
$^1$H-NMR ([$D_6$]-DMSO, δ, ppm):
10,85 (s, br, 1H, N*H*); 8,98 (s, 2H, O*H*); 8,17 (s, 2H, O*H*); 7,65 (d, 2H, Benzylaromat); 7,43 (m, 3H, Benzylaromat) 6,53 (t, 2H, Brenzkatechin); 6,43,-6,38 (m, 4H, Brenzkartechin); 4,39 (s, br, 2H, $C_6H_5$*CH*$_2$N); 3,97 (t, br, $CH_2$*CH*$_2$O); 3,27 (t, br, N*CH*$_2CH_2$); 1,92 (q, br, 4H, $CH_2$*CH*$_2CH_2$)
$^{13}$C-NMR ($CD_3OD$, δ, ppm):
148,3, 147,0, 135,7 (Brenzkatechin); 132,2, 131,1, 130,4, 130,4 (Benzylaromat); 120,3, 110,6, 106,6 (Brenzkatechin); 67,9 ($CH_2$*C*$H_2$O); 58,5 ($C_6H_5$*C*$H_2$N); 52,5 (N*C*$H_2CH_2$); 24,8 ($CH_2$*C*$H_2CH_2$)

***Bis[3-(2,3-dihydroxyphenoxy)propyl]aminhydrochlorid,*** **13**

10 g (21 mmol) **12**, werden in 300 ml absoluten Methanol gelöst und mit 2 g Pd(OH)$_2$/C (20 %) versetzt. In einer Hydrierungsanlage wurde die Mischung unter einem Wasserstoffdruck von 3 bar sechs Stunden bei Raumtemperatur geschüttelt. Der Katalysator wird abfiltriert und das Lösungsmittel abgezogen. Der ölige Rückstand wird 24 h bei 50 °C im Hochvakuum getrocknet.
Ausbeute: 7 g (70 %)
$^1$H-NMR ([$D_5$]-Pyridin, δ, ppm):
8,96 (s, 6H, O*H* und N*H*$_2$); 6,90-6,30 (m, 6H, Brenzkatechin); 3,97 (t, 4H, $CH_2$*CH*$_2$O); 3,17 (t, 4H, N*CH*$_2CH_2$); 2,28 (q, 4H, $CH_2$*CH*$_2CH_2$)
$^{13}$C-NMR ([$D_5$]-Pyridin, δ, ppm):
148,8, 148,2, 136,5, 119,7, 111,0, 106,0 (Brenzkatechin); 67,6 ($CH_2$*C*$H_2$O); 46,5 (N*C*$H_2CH_2$); 26,8

($CH_2$*$CH_2$*$CH_2$)

**Beispiel 3:**

***Bis[3-(2,2-dimethyl-1,3-benzodioxol-4-yloxy)propyl]amin,*** **14**

17,9 g (34,5 mmol) **11** werden in einem Hydrierungskolben in 300 ml absoluten Methanol gelöst, mit 2,0 g (2,8 mmol) des Katalysators ($Pd(OH)_2/C$) versetzt und vier Stunden in einer Wasserstoffhydrierungsapparatur bei 3 bar $H_2$-Druck und 25 ° C geschüttelt. Danach wird der Katalysator abfiltriert und das Lösungsmittel, sowie entstandenes Toluol im Wasserstrahlvakuum entfernt. Das erhaltene Öl wird bei 60 ° C/0,05 mbar sechs Stunden getrocknet.
Ausbeute: 12,9 g (87 %)
$^1$H-NMR ($CDCl_3$, $\delta$, ppm):
6,69 (dd, 2H, Brenzkatechin); 6,46 (d, 2H, Brenzkatechin); 6,43 (d, 2H, Brenzkatechin); 4,14 (t, 4H, $CH_2$*$CH_2$*O); 2,83 (t, 4H, N*$CH_2$*$CH_2$); 1,99 (q, 4H, $CH_2$*$CH_2$*$CH_2$); 1,68 (s, 12H, C(*$CH_3$*)$_2$)
$^{13}$C-NMR ([$D_6$]-Benzol, $\delta$, ppm):
149,3, 143,9, 136,2, 121,6 (Brenzkatechin); 117,9 (*C*($CH_3$)$_2$); 109,1, 102,6 (Brenzkatechin); 67,9 ($CH_2$*$CH_2$*O); 46,6 (N*$CH_2$*$CH_2$); 29,8 ($CH_2$*$CH_2$*$CH_2$); 25,7 (C(*$CH_3$*)$_2$)

***Bis[3-(2,3-dihydroxyphenoxy)propyl]aminhydrochlorid,*** **13**

8 g (18,6 mmol) **14** werden in 80 ml Eisessig gelöst und in der Siedehitze innerhalb von zwei Stunden mit 80 ml eines Säuregemisches (50 % Eisessig, 30 % Wasser, 20 % rauchende Salzsäure) versetzt. Dabei destilliert Lösungsmittel mit frei werdendem Aceton als Azeotrop ab. Nach beendeter Zugabe wird noch eine halbe Stunde destilliert. Danach wird restliches Lösungsmittel am Rotationsverdampfer abgezogen. Der verbleibende Rückstand wird bei 60 ° C/0,05 mbar sechs Stunden im Hochvakuum getrocknet. Das pulverige Produkt wird mit Ether gewaschen. Etherreste werden anschließend an der Ölpumpe entfernt.
Ausbeute: 6,25 g (87 %)
Die Analysendaten sind identisch zu denen von **13** aus **12**.

**Beispiel 4:**

***2,3-Dinitrophenol,*** **15**

15,0 g (108 mmol) 3-Nitrophenol werden in 150 ml Ethanol gelöst. Dazu gibt man 30 g (124,5 mmol) $Cu(NO_3)_2 \cdot 3H_2O$. Die Reaktionsmischung wird danach 20 h unter Rüchfluß zum Sieden erhitzt. Das Lösungsmittel wird am Rotationsverdampfer abgezogen. Man löst den festen Rückstand in 2 M HCl und extrahiert vier mal mit je 50 ml Ether. Die vereinigten Etherextrakte werden über $Na_2SO_4$ getrocknet und vom Lösungsmittel befreit. Der orange Feststoff (21 g) wird über eine kurze Säule an ca. 60 g Kieselgel mit Petrolether/Ether chromatographiert. Zuerst eluiert man 3,6-Dinitrophenol, gefolgt von 3-Nitrophenol und 3,4-Dinitrophenol. Zuletzt erhält man das gewünsche 2,3-Dinitrophenol. Das gewünschte Produkt läßt sich aus Benzol/Petrolether (7:93, v:v) umkristallisieren.
Ausbeute: 2,9 g (14,6 %)
Fp.: 146 ° C
$^1$H-NMR ($CDCl_3$, $\delta$, ppm)
7,18-7,79 (m, 3H, Ar*H*); 9,90 (s, br, 1H, Ar-O*H*)
$^{13}$C-NMR ($CD_3OD$, $\delta$, ppm)
151,87 (*C*-OH); 142,17 (*C*-$NO_2$(m)); 134,22 (*C*-$NO_2$(o)); 132,27 (*C*-H); 124,54 (*C*-H); 116,12 (*C*-H)

***Benzyl-bis[(3-(2,3-dinitrophenoxy)propyl]amin,*** **16**

0,92g (5 mmol) 2,3-Dinitrophenol werden in 10 ml Ethanol gelöst. Dazu gibt man unter Argon 0,28 g (5 mmol) KOH in 25 ml Ethanol. Dabei fällt das Kaliumsalz als roter Feststoff aus. Man gibt 0,65 g (2,5 mmol) Benzyl-bis(3-chlorpropyl)amin, **10**, in 5 ml Ethanol dazu, wobei sich der Feststoff teilweise auflöst. Beim folgenden Erhitzen auf die Siedetemperatur des Ethanols löst sich der Feststoff vollständig auf. Die rote Lösung wird nun 24 h unter Rückfluß gekocht. Es bildet sich ein Niederschlag von KCl. Die Suspension wird heiß gefiltert. Beim Abkühlen kristallisiert **16** in Form farbloser Federn aus. Das Produkt wird aus Ethanol umkristallisiert.

Ausbeute: 0,76 g (55 %)
Fp.: 98-104 ° C
$^1$H-NMR ($CDCl_3$, δ, ppm):
1,91 (q, 4H, $CH_2$*$CH_2$*$CH_2$); 2,60 (t, 4H, N*$CH_2$*); 3,58 (s, 2H, $C_6H_5$*$CH_2$*); 4,15 (t, 4H, O*$CH_2$*); 7,21 (s, 5H, Benzyl-*H*); 7,22-7,81 (m, 6H, Brenzkatechin-*H*)
$^{13}$C-NMR ($CDCl_3$, δ, ppm):
26,70 ($CH_2$*$CH_2$*$CH_2$); 49,81 (N*$CH_2$*); 58,77 ($C_6H_5$*$CH_2$*); 68,57 (O*$CH_2$*); 116,08, 119,56 (*CH*(Phenol)); 126,89, 128.20, 128,74 (*CH*(Benzyl)); 131,04 (*CH*(Phenol)); 134,99 (*C*($NO_2$-o)); 139,06 (*C*(Benzyl)); 140,59 (*C*($NO_2$-m)), 151,37 (*C*(O-Phenol))

***Benzyl-bis[3-(2,3-diaminophenoxy)propyl]amin,* 17**

1,06 g Zinn (8,93 mmol) werden zu 5 ml konzentrierter Salzsäure gegeben. Dazu spritzt man eine Lösung von 0,5 g (0,9 mmol) **16** in 5 ml Methanol. Die Reaktionsmischung wird 30 min auf 50 ° C erwärmt, wobei sie sich braun färbt. Nach dieser Zeit gießt man die Reaktionsmischung zu einer Lösung von 2,5 g NaOH in 50 ml Wasser. Diese Mischung wird fünf mal mit je 15 ml Ether extrahiert. Die Etherextrakte werden mit Wasser gewaschen und über $Na_2SO_4$ getrocknet. Nach dem Abziehen des Ethers verbleibt ein bräunliches Öl, das nicht weiter gereinigt wurde.
Ausbeute: 0,275 g (70 %)
$^1$H-NMR ($CDCl_3$, δ, ppm):
1,99 (q, 4H, $CH_2$*$CH_2$*$CH_2$); 2,69 (t, 4H, N*$CH_2$*); 3,32 (s, 8H, *$NH_2$*); 3,65 (s, 2H, Benz-*$CH_2$*); 4,04 (t, 4H, O*$CH_2$*); 6,28-6,80 (m, 6H, Phenol-*H*); 7,31 (s, 5H, Benz-*H*)
$^{13}$C-NMR ($CD_3Cl$, δ, ppm):
27,14 ($CH_2$*$CH_2$*$CH_2$); 50,24 (N*$CH_2$*); 58,69 ($C_6H_5$*$CH_2$*); 62,23 (O*$CH_2$*); 103,38, 109,50, 118,99 (*CH*(Phenol)); 123,67 (*C*($NH_2$-o)); 126,69, 128,06, 128.59 (*CH*(Benzyl)); 135,32 (*C*($NH_2$-m)); 139,59 (*C*(Benzyl)); 147,71 (*C*-(O-Phenol))

***Bis[3-(2,3-diaminophenoxy)propyl]amin,* 18**

846 mg (1,66 mmol) des Liganden **17** werden in 50 ml Methanol gelöst. Dazu gibt man 0,09g $Pd(OH)_2$ -(auf Kohle, 10 %) und 5 ml Hydrazinhydrat (80 % in Wasser). Die Reaktionsmischung wird 10 h zum Sieden erhitzt. Die resultierende Suspension wird gefiltert und zur Trockne eingeengt. Das Produkt ist in Methanol löslich und fällt nach Zugabe von Diethylether als leicht grünliches Öl aus. Dieses Öl wird an der Ölpumpe von Lösungsmittelresten befreit, wobei man ein graues Pulver erhält.
Ausbeute: 400 mg (69 %)
$^1$H-NMR ($CD_3OD$, δ, ppm):
1,99 (q, 4H, $CH_2$*$CH_2$*$CH_2$); 2,89 (t, 4H, N*$CH_2$*); 4,05 (t, 4H, O*$CH_2$*); 6,24-6,68 (m, 6H, Ar-*H*)
$^{13}$C-NMR ($CD_3OD$, δ, ppm):
29,77 ($CH_2$*$CH_2$*$CH_2$); 47,06 (N*$CH_2$*); 67,70 (O*$CH_2$*); 104,39, 111,13, 120,08 (*CH*(Phenol)); 124,47 (*C*($NH_2$-o)); 136,70 (*C*($NH_2$-m)), 148,87 (*C*(O-Phenol))

**Beispiel 5:**

***Tris[3-(2,3-dinitrophenoxy)propyl]amin,* 19**

1 g 2,3-Dinitrophenol **15** (5,43 mmol) werden mit 0,446 g Tris(3-choroppropyl)amin, **4**, (1,81 mmol) und 0,305 g KOH (5,43 mmol) in 50 ml Ethanol 10 h unter Rückfluß gerührt. Danach läßt man die Reaktionsmischung abkühlen und filtriert den ausgefallenen Feststoff ab. Der Feststoff wird in Aceton aufgenommen. Dabei lösen sich organische Bestandteile, während gebildetes KCl zurückbleibt. Unlösliche Bestandteile werden durch Filtration abgetrennt. Die Aceton-Lösung wird auf 20 ml eingeengt und auf 4 ° C abgekühlt. Nach 24 h kann **19** in Form farbloser Nadeln isoliert werden.
Ausbeute: 638 mg (51 %)
Fp.: 145-147 ° C
$^1$H-NMR ([$D_6$]-Aceton, δ, ppm):
1,93 (q, 6H, $CH_2$*$CH_2$*$CH_2$); 2,59 (t, 6H, N*$CH_2$*); 4,31 (t, 6H, O*$CH_2$*); 7,63-7,88 (m, 9H, Ar-*H*)

***Tris[3-(2,3-diaminophenoxy)propyl]amin,* 20**

638 mg **19** (0,925 mmol) werden zu einer Mischung aus 5 ml HCl (konz.), 5 ml Methanol und 1,64g (13,8 mmol) Zinn gegeben. Die Reaktionsmischung wird 2 h unter Rückfluß gerührt. Die Reaktion ist beendet, wenn sich das Zinn vollständig aufgelöst hat. Nach dem Abkühlen wird die grüne Reaktionslösung mit einem Überschuß an KOH stark alkalisch gemacht (pH 13) und 2 mal mit je 15 ml Aceton ausgeschüttelt. Die vereinigten Aceton-Extrakte werden mit 5 ml Wasser versetzt und zweimal mit je 30 ml Ether ausgeschüttelt. Der Ether-Extrakt wird über Natriumsulfat getrocknet. Das Lösungsmittel wird im Pumpenvakuum entfernt. Man erhält ein gelbliches Öl, das sich beim Trocknen im Pumpenvakuum verfestigt.
Ausbeute: 638 mg (51 %)
$^1$H-NMR ($CDCl_3$, δ, ppm):
1,92 (q, 6H, $CH_2$*$CH_2$*$CH_2$); 2,65 (t, 6 H, N*$CH_2$*); 3,36 (s, 12 H, N*$H_2$*); 3,97 (t, 6 H, O*$CH_2$*); 6,23-6,74 (m, 9H, Ar-*H*)

**Beispiel 6:**

***Darstellung der Technetium-komplexe von* 12 und 13**

Es werden Methanollösungen der Liganden **12** und **13** (30 mmol/l) hergestellt. 8 µl einer solchen Lösung versetzt man mit 10-40 µl einer Saline-Lösung aus einem $^{99m}Tc/^{99}Mo$ Reaktor. Die resultierende Lösung wird sofort dünnschichtchromatographisch untersucht. Diese Untersuchung (Laufmittel THF) zeigt einen vollständigen Einbau des $^{99m}$Tc in den Liganden. Die $R_f$-Werte betragen für $^{99m}TcO_4^-$0,3, für $^{99m}$Tc•**12** 0,65 und für $^{99m}$Tc•**13** 0,60. Reste von Pertechnetat lassen sich bei diesen unterschiedlichen $R_f$-Werten leicht entdecken, werden aber nicht gefunden.

**Beispiel 7:**

***Darstellung des Technetium-Komplexes von* 17**

Man stellt sich eine Methanollösung von **17** mit der Konzentration 50 mmol/l her. Ein µl dieser Lösung wird mit 50 µl einer Eluatlösung eines $^{99m}Tc/^{99}Mo$ Generators gemischt. Dazu gibt man 10 µl 0,1 N NaOH und 10 µl Phosphatpuffer (Ionenstärke 0,1, pH = 7). Die Reaktionsmischung wird 5 min bei Raumtemperatur stehengelassen und anschließend dünnschichtchromatographisch charakterisiert (Laufmittel THF). Diese Analyse zeigt vollständigen Einbau des $^{99m}$Tc in den Liganden. Die $R_f$-Werte betragen für $^{99m}TcO_4^-$0,15, für $^{99m}$Tc•**17** 0,24.

**Beispiel 8:**

***Biotin-NHS***

Zu einer Lösung aus 2,0 g (8,19 mmol) D(+)Biotin und 1,23 g (10,66 mmol) N-Hydroxysuccinimid in 25 ml DMF werden 1,72 g (8,19 mmol) DCCI gegeben. Die entstehende Suspension wird 24 h bei Raumtemperatur gerührt. Der Feststoff wird abfiltriert und die Lösung 4 h auf -16° C gekühlt. Vom ausgefallenen Feststoff wird abfiltriert und das Lösungsmittel des Filtrats im Vakuum abgezogen. Der farblose Rückstand wird mehrmals mit Ether gewaschen und schließlich getrocknet.
Ausbeute: 2,47 g (89 %)
$^1$H-NMR ([$D_6$]-DMSO, δ, ppm, 400 MHz):
6,43, 6,37 (s, br, 2H, b und g); 4,29 (m, br, 1H, c); 4,13 (m, br, 1H, f); 3,09 (m, br, 1H, e); 2,82 (dd, J = 12 Hz, 6 Hz, 1h, d); 2,80 (s, 4H, n); 2,66 (t, J = 7 Hz, 2H, k); 2,57 (d, J = 13 Hz, 1H, d); 1,63-1,50 (m, 6H, h-j);
$^{13}$C-NMR ([$D_6$]-DMSO, δ, ppm):
170,1 (2C, m); 168,8 (l); 162,6 (a); 60,9 (f); 59,1 (c); 55,1 (e); 39,8 (d); 29,9 (k); 27,7, 27.5 (i und j); 25,4 (2C, n); 24,2 (h)

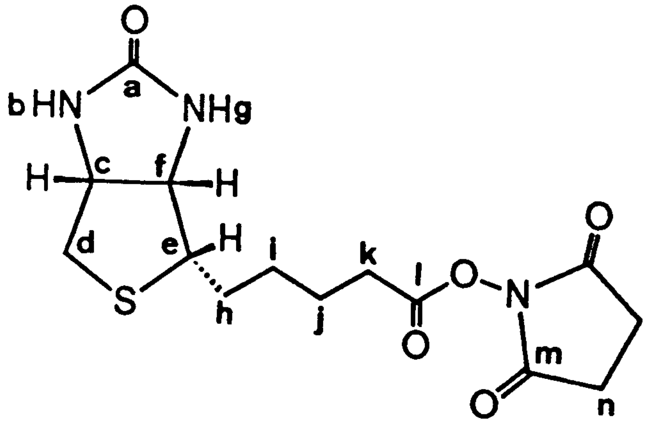

Schema für die Zuordnung der NMR-Signale für Biotin-NHS.

***Kopplung von Biotin-NHS an Bis[3-(2,2-dimethyl-1,3-benzodioxol-4-yloxy)propyl]amin***

860 mg (2,0 mmol) der Ligandenvorstufe **14** werden in 30 ml entgastem DMF mit 670 mg (2 mmol) Biotin-NHS und 860 mg (8 mmol) Triethylamin 80 h bei Raumtemperatur gerührt. Danach wird die Reaktionsmischung mit 80 ml entgastem Wasser versetzt und 1 h auf 4 ° C abgekühlt. Der ausgefallene Feststoff wird durch Filtration isoliert und in 50 ml Aceton aufgenommen. Nach dem Abziehen des Lösungsmittels erhält man das Konjugat als zähflüssiges Öl. Durch Trocknen am Ölpumpenvakuum wird ein voluminöses weißes Pulver erhalten.
Ausbeute: 1,0 g (76 %)
$^1$H-NMR ([$D_6$]-Aceton, $\delta$, ppm, 400 MHz):
6,70 und 6,69 (t, 2H, r und r'); 6,54 und 6,52 (dd, 2H, s und s'); 6,43 und 6,41 (d, 2H, q und q'); 6,23 (s, 1H, b); 5,96 (s, 1H, g); 4,46 (m, 1H, c) 4,27 (m, 1H, f); 4,11 und 4,07 (t, 4H, o und o'); 3,57 und 3,51 (t, 4H, m und m'); 3,15 (m, 1H, e); 2,89 (dd, 1H, d); 2,67 (d, 1H, d); 2,38(t, 2H, k); 1,90 (m, 6H, n, n' und h); 1,63 und 1,62 (s, 12H, w, x, w' und x'); 1,40 (m, 4H, i und j)

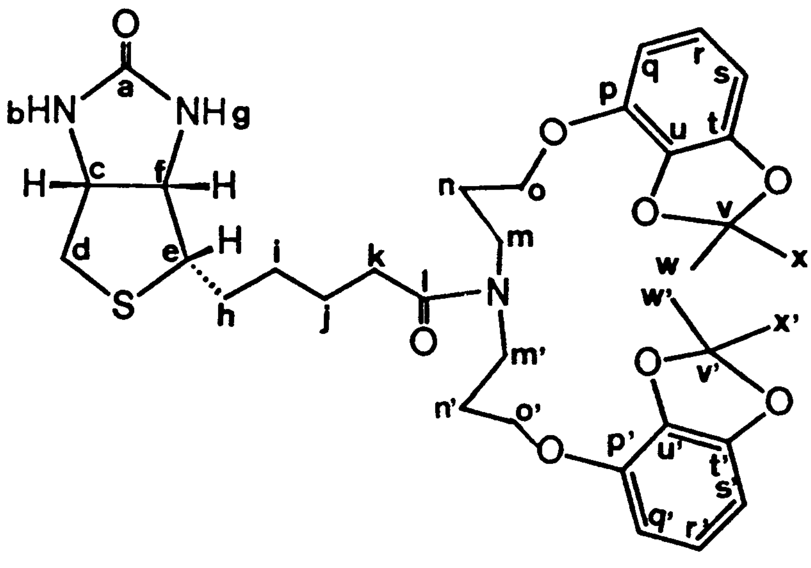

Schema für die Zuordnung des NMR-Signale für das Biotin-**14**-Konjugat.
$^{13}$C-NMR ($CD_3OD$, $\delta$, ppm):
175,6 (l); 165,0 (a); 150,0 und 150,1 (p und p'); 144,2 und 143,9 (t und t'); 136,8 (2C, u und u') 122,6 und 122,5 (r und r'); 119,2 und 119,1 (v und v'); 110,3 (2C, s und s'); 103,6 und 103,4 (q und q'); 68,6 und 67,4 (o und o'); 63,3 (f); 61,5 (c); 56,9 (e); 46,9 und 44,3 (m und m'); 41,0 (d); 33,7 (k); 29,9, 29,7 und 29,5 (h, i

und j); 28,8 und 26,5 (n und n'); 25,9 (4C, w, x, w' und x')

***Abspaltung der Schutzgruppen vom Konjugat Biotin-14***

1 g des Biotin-14 Konjugates wird in 20 ml Methanol gelöst. Dazu gibt man 4 ml rauchende HCl und rührt bei Raumtemperatur 3 Tage. Die Lösungsmittel werden in Vakuum entfernt und der feste Rückstand wird in Methanol gelöst. Die Reinigung erfolgt chromatographisch an $SiO_2$ mit Methanol/THF (1:1) als Laufmittel. Im $^1$H-NMR Spektrum erkennt man die Abspaltung der Schutzgruppen an der Abwesenheit der Signale für die Acetaleinheit. Diese Beobachtung wird auch durch das $^{13}$C-NMR Spektrum bestätigt.
Ausbeute 53 %
Wegen der Probleme bei der Abspaltung der Schutzgruppe wurde noch ein alternativer Syntheseweg untersucht.

**Beispiel 8a:**

***Tetra[dimethyl(tert-butyl)]silylether von* 13**

In einem 100 ml Schlenkkolben werden 3,85 g (10 mmol) **13** und 6,55 g (96 mmol) Imidazol in 35 ml DMF vorgelegt und unter Schutzgas gesetzt. Anschließend wird festes TBDMSCl (7,5 g 50 mmol) zugegeben, wobei Erwärmung eintritt. Es wird 20 h bei Raumtemperatur gerührt. Danach wird die Lösung mit 200 ml Ether versetzt. Diese Mischung wird dreimal mit Wasser gewaschen (Entfernung des DMF) und über Natriumsulfat getrocknet. Nach dem Abfiltrieren des Trockenmittels wird der Ether am Rotationsverdampfer abgezogen. Es verbleibt eine hellgrüne Flüssigkeit. Aus dieser läßt sich das Produkt säulenchromatographisch an $SiO_2$ isolieren (Pentan:Ether 1:2).
Ausbeute: 7 g (86 %)
$^1$H-NMR ($CDCl_3$, $\delta$, ppm):
6,71 (t, 1H, Brenzkatechin); 6,50 (d, 1H, Brenzkatechin); 6,48 (d, 1H, Brenzkatechin); 3,99 (t, 2H, $CH_2C\mathit{H_2}O$); 2,81 (t, 2H, $NC\mathit{H_2}CH_2$); 2,01 (q, 2H, $CH_2C\mathit{H_2}CH_2$); 1,01 und 0,97 (s, 18H, $((C\mathit{H_3})_3C$-SiO); 0,21 und 0,14 (s, 12H, $(C\mathit{H_3})_2$-SiO)
$^{13}$C-NMR ($CDCl_3$, $\delta$, ppm):
151,9, 148.0, 136,3, 120,2, 113,7, 105,9 (Brenzkatechin); 66,8 ($CH_2\mathit{C}H_2O$); 46,9 ($N\mathit{C}H_2CH_2$); 30,0 ($CH_2\mathit{C}H_2CH_2$); 26,1 und 26,0 (6C, $(\mathit{C}H_3)_3CSiO$); 18,6 (2C, $(CH_3)_3\mathit{C}SiO$); -3,8 und -4.0 (4C,$(\mathit{C}H_3)_2SiO$)
MS (70 eV):
806 (2) $M^{+\bullet}$; 749 (23) $M^{+\bullet}$-$C(CH_3)_3$; 452 (100) $M^{+\bullet}$-$C_6H_3(OH)(OSi(CH_3)_2C(CH_3)_3)_2$

***Kopplung des Tetra[dimethyl(tert-bytyl)]silylethers von* 13 *an Biotin***

1,64 g (4.84 mmol) Biotin-NHS und 3,90 g (4,84 mmol) Tetra[dimethyl(tert.-butyl)]silylether von **13** werden mit 2,02 g (20 mmol) Triethylamin in 70 ml DMF 3 d unter Argon gerührt. Das Lösungsmittel wird im Ölpumpenvakuum abgezogen und das verbleibende Öl wird mehrmals mit Wasser gewaschen. Das so erhaltene Rohprodukt wird chromatographisch ($SiO_2$, Methanol) gereinigt. Die Identität des Produktes konnte mitttels der Kernresonanzspektroskopie bewiesen werden.
Ausbeute 43 %

***Abspaltung der Schutzgruppen vom Konjugat Biotin-Tetra[dimethyl(tert-butyl)]silylether von* 13**

1,03 g (1 mmol) (TBDMS)-**13**-Biotin Konjugat werden 24 h in einer Mischung aus 2 ml HCl (konz.) und 20 ml THF gerührt. Danach versetzt man mit 20 ml Wasser und entfernt das THF im Vakuum. Das Konjugat fällt dabei aus. Die salzsaure Wasserlösung wird abdekantiert und der Rückstand wird mehrmals mit Wasser gewaschen. Der Rückstand kann aus Methanol umkristallisiert werden.
Ausbeute 45 %
$^1$H-NMR ($CDCl_3$, $\delta$, ppm):
6,70-6,20 (m, 6H, Brenzkatechin); 4,25 (m, 1H, NH-$C\mathit{H}$-$CH_2$); 4,15 (m, 1H, $CH_2$-$C\mathit{H}$-CH); 3,92 (m, nicht aufgelöst, 4H, O-$C\mathit{H_2}$-$CH_2$); 3,48 (m, nicht aufgelöst, 4H, N-$C\mathit{H_2}$-$CH_2$); 3,10 (m, 1H, CH-$C\mathit{H}$(R)-S); 2,75 (d, 1H, CH-$C\mathit{H}$(H)-S); 2,61 (sbr, 1H, CH-$C\mathit{H}$(H)-S); 2,28 (tbr, 2H, NC(O)-$C\mathit{H_2}$-$CH_2$); 1,95 (m, nicht aufgelöst, 4H, $CH_2$-$C\mathit{H_2}$-$CH_2$-O); 1,55-1,30 (m, 6H, Biotin-Alkylkette)

**Beispiel 9:**

***tert-Butylessigester von* 14**

2,7 g (6.3 mmol) **14** werden in 40 ml THF/$H_2O$ (9:1) gelöst und mit 0,67g (1 Äq.) $Na_2CO_3$ versetzt. Hinzu gibt man 2,46 g (2 Äq.) Bromessigsäure-*tert*.-butylester und rührt bei Raumtemperatur 24 h. Dann gibt man 80 ml $CH_2Cl_2$ hinzu und trocknet über $MgSO_4$. Nach dem Abfiltrieren des Trockenmittels wird die Lösung scharf eingeengt. Der Rückstand wird mehrmals in Hexan aufgenommen und abdekantiert. Das Rohprodukt wird säulenchromatographisch gereinigt (70 g $SiO_2$/Pentan/ Ether 3:1).
Ausbeute: 3,3g (95 %)
$^1$H-NMR ($CDCl_3$, $\delta$, ppm):
6,90-6,30 (m, 6H, Brenzkatechin); 4,11 (t, 4H, $OCH_2CH_2$); 3,26 (s, 2H, OC(O)$CH_2$N); 2,81 (t, 4H, $NCH_2CH_2$); 1,93 (q, 4H, $CH_2CH_2CH_2$); 1,69 (s, 12H, C($CH_3$)$_2$); 1,45 (s, 9H, ($CH_3$)$_3$COC(O))

***Gly*-13**

3,10 g (5.7 mmol) des Produktes der vorherigen Reaktion werden in 50 ml Methanol in der Siedehitze mit 50 ml Säuremischung analog zur Darstellung von **13** aus **12** behandelt. Nach Beendigung der Reaktion wird das Lösungsmittel am Rotationsverdampfer abgezogen. Hierbei fällt das Produkt aus, so daß die wässrige Phase abdekantiert werden kann. Das Produkt wird in Aceton gelöst und im Vakuum vom Lösungsmittel befreit. Dabei fällt es als weißes Pulver an.
Ausbeute 1,90 g (75 %)
$^1$H-NMR ($CDCl_3$, $\delta$, ppm):
6,90-6,30 (m, 6H, Brenzkatechin); 4,17 (t, 4H, O-$CH_2$-$CH_2$); 3,80 (t, 4H, $NCH_2CH_2$); 3,60 (s, 2H, HOOC$CH_2$N); 2,5 (q, 4H, $CH_2CH_2CH_2$)

***Darstellung des NHS-Esters von Gly*-13**

Zu einer Lösung von 1 g (2,5 mmol) Gly-**13** und 0,338 g (2,4 mmol) N-Hydroxysuccinimid in 8 ml DMF wird 0,464 g (2,25 mmol) DCCI gegeben. Nach 24 h rühren bei Raumtemperatur wird der ausgefallene Feststoff abfiltriert und das Filtrat 4 h auf 4° C abgekühlt. Der zusätzlich ausgefallene Feststoff wird wieder abfiltriert und das Filtrat im Vakuum vom Lösungsmittel befreit. Der Rückstand wird einige Male mit Ether nachgewaschen und im Hochvakuum getrocknet.
Ausbeute: 0,95 g (78 %)
$^1$H-NMR ($CDCl_3$, $\delta$, ppm):
6,80-6,20 (m, 6H, Brenzkatechin); 4,13 (t, 4H, $OCH_2CH_2$); 3,80 (s, 2H, OC(O)$CH_2$N); 3,58 (t, 4H, $NCH_2CH_2$); 2,80 (s, 4H, C(O)$CH_2CH_2$C(O)); 2,07 (q, 4H, $CH_2CH_2CH_2$)

**Beispiel 10:**

***4-Nitrophenyl-bis[(3-(2,2-dimethyl-1,3-benzodioxol-4-yloxy)propyl]amin,* 21**

2,15 g (5 mmol) **14** und 0,5 g (5 mmol) Triethylamin werden in 10 ml Ethanol vorgelegt und mit 1,41 g (10mmol) 4-Fluornitrobenzol versetzt. Die Mischung wird 3 Tage gerührt. Danach wird das Lösungsmittel entfernt und das Rohprodukt chromatographisch gereinigt.
Ausbeute: 1,50 g (55 %)
$^1$H-NMR ($CDCl_3$, $\delta$, ppm):
8,06 (d, 2H, Nitrophenyl); 6,72 (t, 2H, Brenzkatechin); 6,71 (d, 2H, Nitrophenyl); 6,47 (d, 2H, Brenzkatechin); 6,46 (d, 2H, Brenzkatechin); 4,13 (t, 4H, $OCH_2CH_2$); 3,67 (t, 4H, $NCH_2CH_2$); 2,11 (q, 4H, $CH_2CH_2CH_2$); 1,71 (s, 12H, C($CH_3$)$_2$)

***4-Aminophenyl-bis[(3-(2,2-dimethyl-1,3-benzodioxol-4-yloxy)propyl]amin,* 22**

1,50 g (2.75 mmol) 4-Nitrophenyl-**14** werden in 30 ml Methanol gelöst und mit 150 mg Pd/C (10 %) unter einer Wasserstoffatmosphäre 4 h bei Raumtemperatur gerührt. Der Katalysator wird abfiltriert und das Lösungsmittel entfernt. Man erhält ein grünliches Öl.
Ausbeute: 1,27 g (89 %)

***4-Aminophenyl-bis-[3-(2,3-dihydroxyohenoxy)propyl]aminhydrochlorid,* 23**

1,04 g (2 mmol) **22**, DIPACE werden unter den Bedingungen zur Darstellung von **13** aus **12** umgesetzt. Man erhält ein weißes Pulver.
Ausbeute: 0,965 g (94 %)

***4-Isothiocyanato-bis[3-(2,3-dihydroxyohenoxy)propyl]aminhydrochlorid,* 24**

0,51 g (1mmol) **23** der vorigen Reaktion werden mit 0,267 g (1.5 mmol) N,N'-thiocarbonyldiimidazol umgesetzt. Nach beendeter Reaktion wird Imidazol mit Wasser herausgewaschen. Man erhält das Produkt als gelbes Öl.
Ausbeute: 360 mg (70 %)

**Beispiel 11:**

***N,N-Bis[3-(2,2-dimethyl-1,3-benzodioxol-4-yloxy)propyl]-N-(2,3-epoxypropyl)amin,* 25**

1,15 g (48 mmol) Natriumhydrid werden in 40 ml DMF bei Raumtemperatur unter Stickstoff suspendiert. Dazu wird eine Lösung von 18,0 g Amin **14** (42 mmol) in 20 ml DMF langsam zugetropft und nach beendeter Zugabe noch 1Stunde gerührt. Anschließend wird eine Lösung von 5,48 g Epibromhydrin (40 mmol) in 20 ml DMF zugetropft und weitere 24Stunden gerührt. Das Gemisch wird mit 70 ml Eiswasser verdünnt, mehrmals mit Ethylacetat extrahiert und die vereinigten organischen Extrakte über Kaliumcarbonat getrocknet. Nach Abziehen des Solvens verbleibt ein gelbes Öl.
Ausbeute: 71%
$^1$H-NMR ($CDCl_3$)
6,90-6,30 (m, 6H, Brenzkatechin); 4,12 (t, 4H, O*CH*2CH2); 3,24 (m, 1H, Epoxid); (2,80-2,60 (m, 8H, N*CH*2 Epoxid); 1,93 (q, 4H, CH2*CH*2CH2); 1,69 (s, 12H, C($CH_3$)$_2$)

***N,N-Bis[3-(2,2-dimethyl-1,3-benzodioxol-4-yloxy)propyl]-N-[2-hydroxy-3-(2-nitroimidazolyl)propyl]amin,* 26**

Eine Mischung aus 250 mg 2-Nitroimidazol (2,2 mmol), 237 mg 1,8-Bis-(dimethylamino)naphthalin (1,1 mmol), 2,12 g **25** (4,4 mmol) und 5 ml DMSO wird unter Feuchtigkeitsausschluß und Rühren 6 Stunden auf 80°C erwärmt, das Solvens im Vakuum abgezogen und der Rückstand chromatographiert (Kieselgel, 230-400 mesh, 3 x 15 cm Säule, 20% $CH_3CN/CHCl_3$ bis 80% $CH_3CN/CHCl_3$). Die einzelnen Fraktionen werden dünnschicht-chromatographisch untersucht und DC-analoge Fraktionen vereinigt. Das Solvens wird abgezogen und der Rückstand im Vakuum getrocknet.
Ausbeute: 27%
$^1$H-NMR ($CDCl_3$)
8,40 (s, 1H, Imidazol); 8,34 (s, 1H, Imidazol); 6,90-6,30 (m, 6H, Brenzkatechin); 4,20-4,00 (m, 5H, O*CH*2CH2, CHOH); 2,90-2,60 (m, 8H, N*CH*2CH2); 1,93 (q, 4H, CH2*CH*2CH2); 1,69 (s, 12H, C($CH_3$)$_2$)

**Beispiel 12:**

***3-{N,N-Bis[3-(2,2-dimethyl-1,3-benzodioxol-4-yloxy)propyl]}aminopropansäureethylester,* 27**

Es werden 0,898 g Kalium-tert-Butylat (8,0 mmol) in 150 ml wasserfreiem tert-Butanol gelöst und eine Lösung von 27,24 g **14** (110 mmol) in 30 ml tert-Butanol und 400 ml Ether zugegeben. Unter Rühren werden 33,04g frisch destillierter Acrylsäureethylester (330 mmol) langsam zugetropft und das Reaktionsgemisch 3 Tage bei RT belassen. Nach Entfernung des Solvens wird das verbleibende Öl in Ether aufgenommen. Die Etherphase wird mit Wasser neutral gewaschen und über Magnesiumsulfat getrocknet. Nach dem Einengen verbleibt ein schwach gelbes Öl.
Ausbeute: 48%
$^1$H-NMR ($CDCl_3$)
6,90-6,30 (m, 6H, Brenzkatechin); 4,25-4,10 (m, 6H, O$CH_2$$CH_2$, $CO_2$$CH_2$$CH_3$); (2,80-2,50 (m, 8H, N$CH_2$$CH_2$,$CH_2$N, CO$CH_2$); 1,93 (q, 4H, $CH_2$$CH_2$$CH_2$); 1,69 (s, 12H, C($CH_3$)$_2$); 1,25 (t, 3H, O$CH_2$$CH_2$)

***3-{N,N-Bis[3-(2,2-dimethyl-1,3-benzodioxol-4-yloxy)propyl]}aminopropansäurehydrazid,* 28**

Zu einer Lösung von 10,0 g des Esters **27** (18,9 mmol) in 200 ml wasserfreiem Pyridin werden 20 g

wasserfreies Hydrazin (624 mmol) gegeben und 3 Tage unter Rückfluß gekocht. Es wird auf 50 ml eingeengt und anschließend mit 200 ml Wasser versetzt, mehrmals mit Ethylacetat extrahiert, die vereinigten organischen Phasen mit Wasser gewaschen, über Natriumsulfat getrocknet und eingeengt. Es verbleibt ein weißer Rückstand.
Ausbeute: 69%
$^1$H-NMR ($CDCl_3$)
6,90-6,30 (m, 6H, Brenzkatechin); 4,08 (t, 4H, O*CH*$_2$CH$_2$); 2,80-2,50 (m, 8H, N*CH*$_2$CH$_2$, *CH*$_2$N, CO*CH*$_2$); 1,93 (q, 4H, CH$_2$*CH*$_2$CH$_2$); 1,69 (s, 12H, C(*CH*$_3$)$_2$);

**Beispiel 13:**

***3-{N,N-Bis[3-(2,2-dimethyl-1,3-benzodioxol-4-yloxy)propyl]}-aminopropansäure, 29***

14,5 g des Esters **27** (27,4 mmol) werden in einer Lösung von 5,00 g Kaliumhydroxid (90,0 mmol) in 75 ml 95%igem Ethanol 2 Stunden unter Rückfluß erhitzt. Das Ethanol wird im Vakuum abgezogen und der verbleibende Rückstand in 100 ml Wasser aufgenommen. Nach Ausschütteln mit 50 ml Ether wird die wässrige Phase mit verdünnter Salzsäure vorsichtig angesäuert. Die freie Säure wird durch mehrmaliges Ausschütteln mit je 50 ml Ether extrahiert. Die vereinigten Etherphasen werden mit gesättigter Kochsalzlösung gewaschen und über Magnesiumsulfat getrocknet. Nach Abziehen des Lösungsmittels verbleibt ein farbloses Öl.
Ausbeute: 85%
$^1$H-NMR ($CDCl_3$)
6,90-6,30 (m, 6H, Brenzkatechin); 4,11 (t, 4H, O*CH*$_2$CH$_2$,); 2,80-2,50 (m, 8H, N*CH*$_2$CH$_2$, *CH*$_2$N, CO*CH*$_2$); 1,93 (q, 4H, CH$_2$*CH*$_2$CH$_2$); 1,69 (s, 12H, C(*CH*$_3$)$_2$)

***3-{N,N-Bis[3-(2,2-dimethyl-1,3-benzodioxol-4-yloxy)propyl]}aminosuccinimidopropionat, 30***

Zu einer auf -5°C gekühlten Lösung von 25,1 g der Carbonsäure **29** (50 mmol) und 5,75 g N-Hydroxysuccinimid (50 mmol) in 100 ml wasserfreiem Tetrahydrofuran wird die Lösung von 12,38 g Dicyclohexylcarbodiimid (60 mmol) in 50 ml Tetrahydrofuran innerhalb von 20 min zugetropft und weitere 2 Stunden bei dieser Temperatur und anschließend noch 15 Stunden bei Raumtemperatur gerührt. Nach Zugabe von 200µl Essigsäure wird eine weitere Stunde gerührt, danach filtriert und der Rückstand zweimal mit heißem Tetrahydrofuran extrahiert. Die vereinigten Filtrate werden zur Trockene eingeengt und der Rückstand aus Ethylacetat umkristallisiert.
Ausbeute: 65%
$^1$H-NMR ($CDCl_3$)
6,90-6,30 (m, 6H, Brenzkatechin); 4,11 (t, 4H, O*CH*$_2$CH$_2$,); 2,80-2,50 (m, 8H, N*CH*$_2$CH$_2$, *CH*$_2$N, CO*CH*$_2$); 2,76 (s, 4H, CO*CH*$_2$*CH*$_2$CO); 1,93 (q, 4H, CH$_2$*CH*$_2$CH$_2$); 1,69 (s, 12H, C(*CH*$_3$)$_2$)

**Beispiel 14:**

***3-{N,N-Bis[3-(2,2-dimethyl-1,3-benzodioxol-4-yloxy)propyl]}aminosuccinimidopropionat, 30***

Zu einer auf 0°C gekühlten Lösung von 25,1 g der Carbonsäure **29** (50 mmol) und 8,30g 2,3,5,6-Tetrafluorphenol (50 mmol) in 100 ml Acetonitril wird die Lösung von 10,37g 1-(3-Dimethylaminopropyl)-3-ethylcarbodiimid (54 mmol) in 100 ml Acetonitril innerhalb von 5 Minuten zugetropft und 2 Stunden auf 75° C erwärmt. Nach Zugabe von 200µl Essigsäure wird eine weitere Stunde gerührt, danach filtriert und der Rückstand zweimal mit heißem Acetonitril extrahiert. Die vereinigten Filtrate werden zur Trockene eingeengt und der Rückstand aus Ethylacetat umkristallisiert.
Ausbeute: 65%
$^1$H-NMR ($CDCl_3$)
6,90-6,30 (m, 7H, Brenzkatechin, Tetrafluorphenol); 4,11 (t, 4H, O*CH*$_2$CH$_2$,); 2,80-2,50 (m, 8H, N*CH*$_2$CH$_2$, *CH*$_2$N, CO*CH*$_2$); 1,93 (q, 4H, CH$_2$*CH*$_2$CH$_2$); 1,69 (s, 12H, C(*CH*$_3$)$_2$)

**Beispiel 15:**

***3-{N,N-Bis[3-(2,2-dimethyl-1,3-benzodioxol-4-yloxy)propyl]}aminopropanol, 32***

Zu einer Suspension von 2,88 g Lithiumaluminiumhydrid (76 mmol) in 150 ml wasserfreiem Ether wird innerhalb einer Stunde die Lösung von 20 g des Esters **27** (38 mmol) in 50 ml wasserfreiem Ether so zugetropft, daß die Lösung mäßig siedet. Danach wird noch 5 Stunden unter Rückfluß erhitzt, auf Raumtemperatur abgekühlt und überschüssiges Hydrid mit Wasser vorsichtig hydrolisiert. Es wird vom ausgefallenden Hydroxid abfiltriert und das Filtrat mehrmals mit warmen Ether gewaschen. Nach Entfernen des Lösungsmittels im Vakuum wird der Rückstand kurz in Ethanol aufgekocht, nochmals filtriert und das Solvens abgezogen. Es verbleibt eine hochviskose Flüssigkeit.
Ausbeute: 61%
$^1$H-NMR ($CDCl_3$)
6,90-6,30 (m, 6H, Brenzkatechin); 4,12 (t, 4H, O*CH2*CH2); 3,54 (t, 2H, *CH2*OH); (2,80-2,60 (m, 6H, N*CH2*); 2,00-1,80 (m, 6H, CH2*CH2*CH2); 1,69 (s, 12H, C(*CH*$_3$)2)

**Beispiel 16:**

***Bis[3-(2,2-dimethyl-1,3-benzodioxol-4-yloxy)propyl]-N-(3-chlorpropyl)amin, 33***

Eine Lösung von 10,1 g des Alkohols **32** (20,7 mmol) in 60 ml wasserfreiem Tetrachlorkohlenstoff wird unter einer Stickstoffatmosphäre mit 7,86 g Triphenylphosphin (30 mmol) versetzt. Es wird mehrere Stunden unter Rückfluß erhitzt. Nach Abkühlung wird mit dem halben Volumen Petrolether verdünnt und einige Zeit bei -20°C aufbewahrt. Der Niederschlag wird abgesaugt und mit Petrolether gewaschen, nach Trocknen über Natriumsulfat und Abziehen des Lösungsmittels verbleibt ein gelbes Öl.
Ausbeute: 78%
$^1$H-NMR ($CDCl_3$)
6,90-6,30 (m, 6H, Brenzkatechin); 4,11 (t, 4H, O*CH2*CH2); 3,62 (t, 2H, *CH2*Cl); (2,80-2,60 (m, 6H, N*CH2*); 2,00-1,80 (m, 6H, CH2*CH2*CH2); 1,66 (s, 12H, C(*CH*$_3$)$_2$)

**Beispiel 17:**

***3-{N,N-Bis[3-(2,2-dimethyl-1,3-benzodioxol-4-yloxy)propyl]}aminopropanal, 34***

Zu einer gut gerührten Suspension von 3,23 g PCC in 25ml wasserfreiem Dichlormethan werden 4,88 g des Alkohols **32** (10 mmol), gelöst in 20 ml Dichlormethan, auf einmal zugefügt und das Gemisch 90 min bei Raumtemperatur gerührt. Nach Zugabe von 50 ml wasserfreiem Ether wird abdekantiert und der Rückstand dreimal mit je 20 ml gewaschen, die vereinigten Etherlösungen werden über 20 g Kieselgel filtriert. Nach Abziehen des Solvens verbleibt ein gelbes Öl.
Ausbeute: 70%
$^1$H-NMR ($CDCl_3$)
9,74 (s, 1H, Aldehyd); 6,90-6,30 (m, 6H, Brenzkatechin); 4,12 (t, 4H, O*CH2*CH2); 2,80-2,60 (m, 6H, N*CH2*); 2,42 (t, 2H, $CH_2$CHO); 2,00-1,70 (m, 6H, CH2*CH2*CH2); 1,66 (s, 12H, C($CH_3$)$_2$)

**Beispiel 18:**

***3-{N,N-Bis[3-(2,2-dimethyl-1,3-benzodioxol-4-yloxy)propyl]}aminopropionsäurenitril, 35***

Es werden 0,90 g Kalium-tert-Butylat (8,0 mmol) in 150 ml wasserfreiem tert-Butanol gelöst und eine Lösung von 27,2 g **14** (110 mmol) in 30 ml tert-Butanol und 400 ml Ether zugegeben. Unter Rühren werden 17,5 g frisch destilliertes Acrylnitril (330 mmol) langsam zugetropft und das Reaktionsgemisch 12 Stunden unter Rückfluß erhitzt. Nach Entfernung des Solvens wird das verbleibende Öl in Ether aufgenommen. Die Etherphase wird mit Wasser neutral gewaschen und über Magnesiumsulfat getrocknet. Nach dem Einengen verbleibt ein schwach gelbes Öl.
Ausbeute: 62%
$^1$H-NMR ($CDCl_3$)
6,90-6,30 (m, 6H, Brenzkatechin); 4,11 (t, 4H, O$CH_2$$CH_2$,); 2,81 (t, 4H, N$CH_2$$CH_2$); 2,50-2,30 (m, 4H, N$CH_2$$CH_2$CN); 1,97 (q, 4H, $CH_2$$CH_2$$CH_2$); 1,69 (s, 12H, C($CH_3$)$_2$)

**Beispiel 19:**

***N,N-Bis[3-(2,2-dimethyl-1,3-benzodioxol-4-yloxy)propyl]propylendiamin, 36***

Zu einer Suspension von 30,6 g Lithiumaluminiumhydrid (0,81 mol) in 500 ml wasserfreiem Ether werden unter Eiskühlung 39,0 g 100% Schwefelsäure (0,40 mol) langsam zugetropft. Anschließend wird eine Stunde bei Raumtemperatur gerührt, dann wird die Lösung von 12,55 g des Nitrils **35** (0,26 mol) in 50 ml wasserfreiem Ether so zugetropft, daß die Lösung mäßig siedet. Danach wird noch 8 Stunden unter Rückfluß erhitzt, auf Raumtemperatur abgekühlt und überschüssiges Hydrid mit Wasser vorsichtig hydrolisiert. Es wird eine Lösung von 40 g NaOH in 360 ml Wasser zugefügt, vom ausgefallenden Hydroxid abfiltriert und das Filtrat mehrmals mit warmen Ether gewaschen. Die vereinigten Etherextrakte werden über Kaliumcarbonat getrocknet und das Solvens abgezogen. Es verbleibt ein gelbes Öl.
Ausbeute: 47%
$^1$H-NMR ($CDCl_3$)
6,90-6,30 (m, 6H, Brenzkatechin); 4,12 (t, 4H, O*CH*2CH2); (2,80-2,50 (m, 8H, N*CH2*); 2,00-1,80 (m, 6H, CH2*CH*2CH2); 1,69 (s, 12H, C($CH_3$)$_2$)

**Beispiel 20:**

***N,N-Bis[3-(2,2-dimethyl-1,3-benzodioxol-4-yloxy)propyl]-N-[4-(nitrobenzyl)]amin, 37***

1,15 g (48 mmol) Natriumhydrid werden in 40 ml DMF bei Raumtemperatur unter Stickstoff suspendiert. Dazu wird eine Lösung von 18,0 g Amin **14** (42 mmol) in 20 ml DMF langsam zugetropft und nach beendeter Zugabe noch 1Stunde gerührt. Anschließend wird eine Lösung von 8,64g 4-Nitrobenzylbromid (40 mmol) in 20 ml DMF zugetropft und weitere 24 Stunden gerührt. Das Gemisch wird mit 70 ml Eiswasser verdünnt, mehrmals mit Ethylacetat extrahiert und die vereinigten organischen Extrakte über Kaliumcarbonat getrocknet. Nach Abziehen des Solvens verbleibt ein gelbes Öl.
Ausbeute: 71%
$^1$H-NMR ([$D_{6]-Aceton}$)
8,21 (d, 2H, Nitroaryl); 7,53 (d, 2H, Nitroaryl); 6,60-6,40 (m, 6H, Brenzkatechin); 4,10 (t, 4H, O*CH*2CH2); 3,63 (s, 2H, C6H5*CH*2N); 2,61 (t, 4H, N*CH*2CH2); 1,91 (q, 4H, CH2*CH*2CH2); 1,60 (s, 12H, C($CH_3$)$_2$)

***N,N-Bis-[3-(2,2-dimethyl-1,3-benzodioxol-4-yloxy)propyl]-N-(4-aminobenzyl)amin, 38***

In einem 500 ml Zweihalskolben werden 200 mg 10% Pd/C in 250 ml Methanol suspendiert, auf -20 °C abgekühlt und mit Wasserstoff gesättigt. Anschließend wird die Lösung von 5,0 g **37** (8,8 mmol) in 50 ml Methanol schnell zugetropft und bei -20°C gerührt. Nach Beendigung der Wasserstoffaufnahme wird vom Katalysator abgetrennt und das Lösungsmittel im Vakuum abgezogen. Es verbleiben schwach gelbe Kristalle.
Ausbeute: 85%
$^1$H-NMR ([$D_{6]-Aceton}$)
7,04 (d, 2H, Aminoaryl); 6,60-6,40 (m, 8H, Aminoaryl, Brenzkatechin); 4,10 (t, 4H, O*CH*2CH2); 3,36 (s, 2H, C6H5*CH*2N); 2,63 (t, 4H, N*CH*2CH2); 1,89 (q, 4H, CH2*CH*2CH2); 1,59 (s, 12H, C($CH_3$)$_2$)

***N,N-Bis[3-(2,3-dihydroxyphenoxy)propyl]-N-(4-aminobenzyl)aminhydrochlorid, 39***

10,7 g (20 mmol) **37** werden in 80 ml Eisessig gelöst und in der Siedehitze innerhalb von zwei Stunden mit 80 ml eines Säuregemisches (50% Eisessig, 30% Wasser, 20% rauchende Salzsäure) versetzt. Dabei destilliert Lösungsmittel mit frei werdendem Aceton als Aceotrop ab. Nach beendeter Zugabe wird noch 30 Minuten destilliert. Danach wird restliches Lösungsmittel am Rotationsverdampfer abgezogen. Der verbleibende Rückstand wird 6h im Hochvakuum getrocknet.
Ausbeute: 79%
$^1$H-NMR ([$D_5$]-Pyridin)
8,93 (s, 6H, OH und $NH_2$); 7,10-6,40 (m, 10H, Aminoaryl, Brenzkatechin); 3,92 (t, 4H, O*CH*2CH2); 3,47 (s, 2H, C6H5*CH*2N); 3,12 (t, 4H, N*CH*2CH2); 2,25 (q, 4H, CH2*CH*2CH2)

***N,N-Bis[3-(2,2-dimethyl-1,3-benzodioxol-4-yloxy)propyl]-N-(4-isothiocyanatobenzyl)amin-hydrochlorid, 40***

Zu einer Lösung von 1,10 g des Anilins **39** (2,23 mmol) in 50 ml 3M Salzsäure und 50 ml Chloroform wird unter einer Stickstoffatmosphäre mit Hilfe einer Einwegspritze 1,15 g Thiophosgen (10 mmol) gegeben und 6 Stunden bei Raumtemperatur intensiv gerührt. Anschließend wird im Vakuum zur Trochne eingeengt.

Ausbeute: 78%
$^1$H-NMR ([$D_5$]-Pyridin)
8,96(s, 4H, OH); 7,10-6,40 (m, 10H, Aryl, Brenzkatechin); 3,92 (t, 4H, O*CH*2CH2); 3,61 (s, 2H, C6H5*CH*2N); 3,21 (t, 4H, N*CH*2CH2); 2,27 (q, 4H, CH2*CH*2CH2)

**Beispiel 21:**

***N,N-Bis[3-(2,2-dimethyl-1,3-benzodioxol-4-yloxy)propyl]-N-(2-propenyl)amin, 41***

1,20 g (50 mmol) Natriumhydrid werden in 50 ml DMF bei Raumtemperatur unter Stickstoff suspendiert. Dazu wird eine Lösung von 20,6 g Amin **14** (48 mmol) in 40 ml DMF langsam zugetropft und nach beendeter Zugabe noch 1Stunde gerührt. Anschließend wird eine Lösung von 6,04g Allylbromid (50 mmol) in 25 ml DMF zugetropft und weitere 24Stunden gerührt. Das Gemisch wird mit 70 ml Eiswasser verdünnt, mehrmals mit Ethylacetat extrahiert und die vereinigten organischen Extrakte über Kaliumcarbonat getrocknet. Nach Abziehen des Solvens verbleibt ein gelbes Öl.
Ausbeute: 75%
$^1$H-NMR ($CDCl_3$)
6,90-6,30 (m, 6H, Brenzkatechin); 5,62 (m, 1H, *CH*=$CH_2$); 4,93 (m, 2H, CH=$CH_2$); 4,09 (t, 4H, O*CH*2CH2); 2,80-2,60 (m, 6H, N*CH*2); 2,32 (m, 2H, $CH_2$CH=$CH_2$); 2,00-1,80 (m, 6H, CH2*CH*2CH2); 1,66 (s, 12H, C-($CH_3$)$_2$)

**Beispiel 22:**

***Bis[3-(2,2-dimethyl-1,3-benzodioxol-4-yloxy)propyl](2-propinyl)amin, 42***

0,24 g (10mmol) Natriumhydrid werden in 50 ml DMF bei Raumtemperatur unter Stickstoff suspendiert. Dazu wird eine Lösung von 4,29g Amin **14** (10 mmol) in 20 ml DMF langsam zugetropft und nach beendeter Zugabe noch 1Stunde gerührt. Anschließend wird eine Lösung von 1,43g Propargylbromid (12 mmol) in 10 ml DMF zugetropft und weitere 24Stunden bei 50°C gerührt. Nach dem Abkühlen wird das Gemisch mit 70 ml Eiswasser verdünnt, mehrmals mit Ethylacetat extrahiert und die vereinigten organischen Extrakte über Kaliumcarbonat getrocknet. Nach Abziehen des Solvens verbleibt ein gelbes Öl.
Ausbeute: 66%
$^1$H-NMR ($CDCl_3$)
6,90-6,30 (m, 6H, Brenzkatechin); 4,15 (t, 4H, O*CH*2CH2); 2,80-2,60 (m, 8H, N*CH*2, $CH_2$CCH); 2,00-1,80 (m, 7H, CH2*CH*2CH2, CH2C*CH*); 1,65(s, 12H, C($CH_3$)$_2$)

**Beispiel 23:**

***Kopplung eines Isothiocyanat-haltigen Tc-99m-Komplexes an Proteine***

Am Beispiel von F(ab')$_2$-Fragmenten des monoklonalen Antikörpers 17-1A soll die Kopplung von Isothiocyanat-haltigen Tc-99m-Komplexen [Beispiel 10] an Proteine beschrieben werden. Anstelle der Antikörper-Fragmente kann jedes andere Protein bzw. eine Aminogruppen-haltige Substanz verwendet werden.

Der monoklonale Antikörper 17-1A wird entsprechend literaturbekannter Methoden nach Applikation von $10^7$ der entsprechenden Hybridomazellen in den Bauchraum einer Balb/c-Maus und Aspiration der Ascitesflüssigkeit nach 7 - 10 Tagen gewonnen. Die Reinigung erfolgt nach ebenfalls literaturbekannten Methoden durch Ammoniumsulfatfällung und Affinitätschromatographie über Protein A-Sepharose. Der gereinigte Antikörper (10 mg/ml) wird bei pH 3,5 für 2 Std. mit 25 $\mu$g/ml Pepsin behandelt und die F(ab')2-Fragmente anschließend mittels FPLC isoliert. Vor der Kopplung mit dem Chelatbildner werden die Fragmente bei 4 °C für 12 - 24 Std. gegen 0,1 M $KH_2PO_4$/0,1 M $NaHCO_3$, pH 8,5, dialysiert. Die Proteinkonzentration wird auf 10 mg/ml eingestellt. Der analog Beispiel 6 markierte NCS-haltige Komplex wird in einem molaren Verhältnis 1 : 10 (Komplex : Protein) zur Proteinlösung hinzugegeben. Zur Konjugatbildung wird die Mischung für 1 Std. bei 37 °C inkubiert.

**Beispiel 24:**

***Bioverteilung eines Tc-99m-Komplexes gekoppelt an Fragmente des monoklonalen Antikörpers 17-***

*1A*

Am Beispiel eines Konjugates mit F(ab')2-Fragmenten des monoklonalen Antikörpers 17-1A soll die Bioverteilung von Protein-gebundenen Tc-99m-Komplexen beschrieben werden. Der Antikörper, aus dem die Fragmente gewonnen werden, erkennt ein Antigen, das von der menschlichen Carcinomzellinie "HT29" exprimiert wird. Eine Kontrollzellinie, die ebenfalls aus einem menschlichen Carcinom (MX-1) gewonnen wurde, exprimiert dieses Antigen nicht. Isolierte Zellen beider Linien werden immundefizienten Nacktmäusen subcutan appliziert. Nachdem die Tumoren zu einer Größe von 300 - 800 mg herangewachsen sind, werden den Mäusen intravenös 20 µg des mit 200 µCi Tc-99m markierten Komplexes gekoppelt an F(ab')-2-Fragmente [Beispiel 11] verabreicht. Die Immunreaktivität der Konjugate wird parallel mittels der Bindung an einen Überschuß von zellständigem Antigen bestimmt und beträgt 75 - 80 %. Die Bioverteilung wird 24 Std. nach Applikation des Konjugates durch Tötung der Tiere, Entnahme der Organe und Messung der Radioaktivität in den Organen bestimmt. Die folgende Tabelle stellt die gefundenen Radioaktivitätsmengen dar und zeigt eine deutliche Anreicherung des Chelats in dem Antigen-positiven Tumor.

| Organ | % der applizierten Dosis pro Gramm Gewebe |
|---|---|
| Milz | 0,4 |
| Leber | 1,1 |
| Nieren | 2,8 |
| Lunge | 0,9 |
| Muskel | 0,1 |
| Blut | 0,6 |
| MX-1 | 1,9 |
| HT29 | 8,8 |

**Beispiel 25:**

***Bioverteilung eines Biotin-haltigen Tc-99m-Komplexes***

20 µl eines kommerziell erhältlichen Streptavidin-gekoppelten Sepharose-Gels (entsprechend 20 µg Streptavidin) werden einer 200 g schweren Ratte in den Muskel des linken Hinterbeins appliziert. Etwa 30 min danach erfolgt die intravenöse Applikation von 5 µg des Biotin-haltigen Komplexes [Beispiel 8] markiert mit 200 µCi Tc-99m entsprechend dem Beispiel 6. Die Bestimmung der Radioaktivität in den einzelnen Organen der Ratte erfolgt nach 4 Stunden. Eine 16fach höhere Radioaktivität, die im linken Hinterlaufmuskel im Vergleich zum rechten Hinterlaufmuskel gefunden wird, zeigt eine deutliche spezifische Anreicherung des Tc-Komplexes durch Bindung an Streptavidin-Sepharose. In allen anderen Organen wird nach 4 Stunden keine Aktivitäten über 1,4 % der applizierten Dosis pro Gramm Gewebe detektiert. Die höchste Anreicherung nach dem linken Hinterlaufmuskel (1,4 % der applizierten Dosis pro Gramm Gewebe) findet sich in den Nieren mit 0,6% der appl. Dosis pro Gramm Gewebe. Etwa 89 % der applizierten Radioaktivität werden nach 4 Stunden im Harn gefunden.

Das Beispiel zeigt, daß die Biotin-haltigen Komplexe im Organismus an Streptavidin-Konjugate binden können. Anstelle eines Streptavidin-Sepharose-Konjugates können selektive Substanzen, wie z.B. monoklonale Antikörper, Enzyme oder Hormone, verwendet werden, die sich - an Streptavidin gekoppelt - nach selektiver Anreicherung in Läsionen oder bestimmten Geweben durch die Biotin-haltigen Tc-99m-Komplexe detektieren lassen.

**Patentansprüche**

**1.** Verbindungen der allgemeinen Formel I,

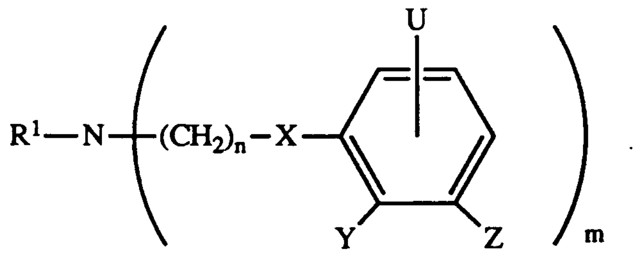

(I)

worin X fur -O-, -S-, $-NR^2-$ mit $R^2$ in der Bedeutung eines Wasserstoffatoms oder eines $C_{1-6}$-Alkylrestes,

Y und Z gleich oder verschieden sind und für die Reste -OH, $-NHR^3$ oder $-SR^3$ mit $R^3$ in der Bedeutung eines Wasserstoffatoms oder eines $C_{1-6}$-Alkylrestes und

U für ein Wasserstoffatom, einen verzweigten oder unverzweigten $C_{1-6}$-Alkyl-, einen $C_{1-6}$-Alkoxy-, einen Hydroxyl- oder einen Carboxylrest stehen,

n die Ziffern 2 bis 6 und
m die Ziffern 2 oder 3 bedeutet und $R^1$ nur vorhanden ist, wenn m für die Ziffer 2 steht,

$R^1$ für ein Wasserstoffatom, einen Benzyl-, oder einen verzweigten oder unverzweigten gegebenenfalls mit ein bis drei Hydroxyl-, Carboxyl- oder Aminogruppen substituierten $C_{0-6}$-Alkylrest steht, der gegebenenfalls eine funktionelle Gruppe B oder eine - gegebenenfalls mit Hilfe dieser funktionellen Gruppe gebundene - sich selektiv in Läsionen oder bestimmten Geweben anreichernde Verbindung T enthält,

wobei B im Falle, daß Y und/oder Z für $NHR^3$ steht, für einen Amino-, einen Hydrazino- oder Hydrazid-, einen Carboxyl-, einen $C_{1-6}$-Alkinyl- oder -Alkenyl-, einen Hydroxyl-, einen Aminophenyl-, einen Oxiranyl-, einen fluorierten Phenoxycarbonyl- oder einen Biotinrest,

oder im Falle, daß Y und Z für -OH oder $SR^3$ steht, zusätzlich auch für einen Halogen-, einen Formyl-, einen Nitril-, einen Phenylisothiocyanato- oder einen gegebenenfalls mit einem Natriumsulfatrest substituierten Succinimidoxycarbonylrest,

und T für monoklonale Antikörper oder deren Fragmente, Hormone, Wachstumsfaktoren, Liganden für Zellmembranrezeptoren, Steroide, Neurotransmitter, Fettsäuren, Sacharide, Aminosäuren und Oligopeptide, Biotin, sowie Radiosensitizer wie z.B. Misonidazol steht,

wobei in $R^1$ gegebenenfalls vorhandene funktionelle Gruppen gegebenenfalls in geschützter Form oder deren Vorstufen vorliegen,

mit Ausnahme der Verbindung $N\{CH_2-CH_2-CH_2-O-C_6H_3-2,3-(OH)_2\}_3$,

deren Technetium- und Rhenium-Komplexe, sowie deren Salze mit anorganischen und organischen Säuren.

**2.** Verbindungen nach Anspruch 1, dadurch gekennzeichnet, daß U ein Wasserstoffatom darstellt.

**3.** Verbindungen nach Anspruch 1, dadurch gekennzeichnet, daß X ein Sauerstoffatom darstellt.

**4.** Verbindungen nach Anspruch 1, dadurch gekennzeichnet, daß n die Ziffer 3 bedeutet.

**5.** Verbindungen nach Anspruch 1, dadurch gekennzeichnet, daß Y und Z gleich sind und für $NH_2$- oder OH- stehen.

6. Verbindungen nach Anspruch 1, dadurch gekennzeichnet, daß m für die Ziffer 2 steht und $R^1$ für ein Wasserstoffatom, einen Benzyl-, oder einen unverzweigten gegebenenfalls mit einer Hydroxyl- oder Aminogruppe substituierten $C_{0-3}$-Alkylrest steht, der gegebenenfalls eine funktionelle Gruppe B oder - mit Hilfe von B gekoppelt - monoklonale Antikörper bzw. deren Fragmente, Steroide oder Misonidazol enthält.

7. Verbindungen nach Anspruch 1, dadurch gekennzeichnet, daß m und n für die Ziffer 3, X für ein Sauerstoffatom, U für ein Wasserstoffatom und Y und Z gleich sind und für $NH_2$- oder OH- stehen.

8. Verwendung der Technetium- und Rheniumchelate gemäß Anspruch 1 für die In-vivo-Diagnostik und Therapie, bevorzugt für die Tumortherapie.

9. Pharmazeutische Mittel enthaltend mindestens ein Technetium- oder Rheniumchelat gemäß Anspruch 1, gegebenenfalls mit den in der Galenik üblichen Zusätzen.

10. Verfahren zur Herstellung von Verbindungen der allgemeinen Formel I,

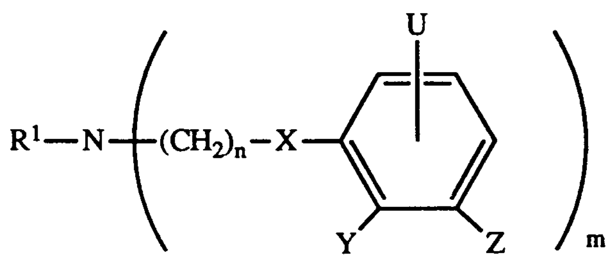

(I)

worin X für -O-, -S-, $-NR^2-$ mit $R^2$ in der Bedeutung eines Wasserstoffatoms oder eines $C_{1-6}$-Alkylrestes,

Y und Z gleich oder verschieden sind und für die Reste -OH, $-NHR^3$ oder $-SR^3$ mit $R^3$ in der Bedeutung eines Wasserstoffatoms oder eines $C_{1-6}$-Alkylrestes und

U für ein Wasserstoffatom, einen verzweigten oder unverzweigten $C_{1-6}$-Alkyl-, einen $C_{1-6}$-Alkoxy-, einen Hydroxyl- oder einen Carboxylrest stehen,

n die Ziffern 2 bis 6 und
m die Ziffern 2 oder 3 bedeutet und $R^1$ nur vorhanden ist, wenn m für die Ziffer 2 steht,

$R^1$ für ein Wasserstoffatom, einen Benzyl-, oder einen verzweigten oder unverzweigten gegebenenfalls mit ein bis drei Hydroxyl-, Carboxyl- oder Aminogruppen substituierten $C_{0-6}$-Alkylrest steht, der gegebenenfalls eine funktionelle Gruppe B oder eine - gegebenenfalls mit Hilfe dieser funktionellen Gruppe gebundene - sich selektiv in Läsionen oder bestimmten Geweben anreichernde Verbindung T enthält,

wobei B im Falle, daß Y und/oder Z für $NHR^3$ steht, für einen Amino-, einen Hydrazino- oder Hydrazid-, einen Carboxyl-, einen $C_{1-6}$-Alkinyl- oder -Alkenyl-, einen Hydroxyl-, einen Aminophenyl-, einen Oxiranyl-, einen fluorierten Phenoxycarbonyl- oder einen Biotinrest,

oder im Falle, daß Y und Z für -OH oder $SR^3$ steht, zusätzlich auch für einen Halogen-, einen Formyl-, einen Nitril-, einen Phenylisothiocyanato- oder einen gegebenenfalls mit einem Natriumsulfatrest substituierten Succinimidoxycarbonylrest,

und T für monoklonale Antikörper oder deren Fragmente, Hormone, Wachstumsfaktoren, Liganden für Zellmembranrezeptoren, Steroide, Neurotransmitter, Fettsäuren, Sacharide, Aminosäuren und Oligopeptide, Biotin, sowie Radiosensitizer wie z.B. Misonidazol steht,

wobei in $R^1$ gegebenenfalls vorhandene funktionelle Gruppen gegebenenfalls in geschützter Form oder deren Vorstufen vorliegen,

mit Ausnahme der Verbindung $N\{CH_2-CH_2-CH_2-O-C_6H_3-2,3-(OH)_2\}_3$,

deren Technetium- und Rhenium-Komplexe, sowie deren Salze mit anorganischen und organischen Säuren,

dadurch gekennzeichnet, daß man Amine der allgemeinen Formel II,

$R^{1'}—N\{—CH_2)_n—Nu\}_m$ (II)

worin Nu für ein Nucleofug wie z.B. Cl, Br, I, $CH_3C_6H_4SO_3$, $CH_3SO_3$ oder $CF_3SO_3$

und $R^{1'}$ für einen Substituenten $R^1$, dessen gegebenenfalls vorhandenen funktionellen Gruppen in geschützter Form oder als deren Vorstufen vorliegen und der keine sich selektiv anreichernde Verbindung T enthält, stehen

mit Aromaten der allgemeinen Formel III

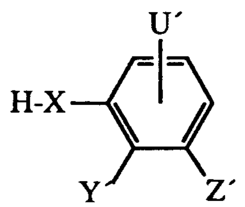

(III)

worin U' für einen Substituenten U, dessen Hydroxy- oder Carboxylrest in geschützter Form vorliegt, und Y' und Z' für Y und Z oder deren Vorstufen oder in geschützter Form stehen,

unter Basenkatalyse in polaren Lösungsmitteln bei Temperaturen von 50 - 200 °C innerhalb von 6 Stunden bis 6 Tagen, vorzugsweise 2 Stunden bis 4 Tage, umsetzt

und anschließend die in $R^1$ gegebenenfalls enthaltene funktionelle Gruppe B oder die gewünschten Aromat-Substituenten Y und Z generiert, gewünschtenfalls die so erhaltenen kopplungs- bzw. komplexierungsfähigen Verbindungen mit der jeweils gewünschten sich selektiv anreichernden Verbindung T koppelt bzw. mit dem jeweils gewünschten Technetium- oder Rhenium-Isotop komplexiert - wobei die Reihenfolge der Schritte Kopplung an T und Komplexierung mit dem Technetium- oder Rhenium-Isotop vertauscht werden kann - und anschließend die noch vorhandenen Schutzgruppen entfernt bzw. die Vorstufen in die letztendlich gewüschten Substituenten überführt.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung

EP 91 25 0221

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl.5) |
|---|---|---|---|
| P,X | ZEITSCHRIFT FÜR NATURFORSCHUNG, Teil B: Band 46B, Nr. 6, 2. Januar 1991, Seiten 779-782, Verlag der Zeitschrift für Naturforschung, Tübingen, DE; F.E. HAHN et al.: "Synthese von Di- und Tri(o-phenylendiamin)-Liganden"<br>* Das ganze Artikel *<br>--- | 1-10 | C 07 C 217/84<br>C 07 C 217/20<br>C 07 F 13/00<br>C 07 D 495/04<br>C 07 C 331/28<br>C 07 K 15/28<br>A 61 K 49/02 |
| P,X | CHEMISCHE BERICHTE, Band 124, Nr. 3, Marz 1991, Seiten 481-486, VCH Verlagsgesellschaft mbH, Weinheim, DE; F.E. HAHN et al.: "99mTc-Komplexe mit sechszähnigen Tribrenzkatechin-Liganden"<br>* Das ganze Artikel *<br>--- | 1-5,7-10 | |
| P,X | CHEMISCHE BERICHTE, Band 124, Nr. 3, Marz 1991, Seiten 487-491, VCH Verlagsgesellschaft mbH, Weinheim, DE; F.E. HAHN et al.: "99mTc-Komplexe mit vierzähnigen Dibrenzkatechin-Liganden"<br>* Das ganze Artikel *<br>--- -/- | 1-6,8-10 | RECHERCHIERTE SACHGEBIETE (Int. Cl.5)<br>C 07 C<br>C 07 F<br>C 07 D<br>C 07 K<br>A 61 K |

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Obwohl Anspruch 8 sich auf ein Diagnostizierverfahren bzw. Verfahren zur Behandlung des menschlichen Körpers bezieht (Art. 52(4) EPÜ) wurde die Recherche duchgeführt und gründete sich auf die angeführten Wirkungen der Verbindung.

| Recherchenort | Abschlußdatum der Recherche | Prüfer |
|---|---|---|
| DEN HAAG | 21-01-1991 | WELLS A.G. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0409)

Europäisches Patentamt

# EUROPÄISCHER TEILRECHERCHENBERICHT

Nummer der Anmeldung
EP 91 25 0221

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. 5) |
|---|---|---|---|
| D,A | JOURNAL OF THE AMERICAN CHEMICAL SOCIETY, Band 111, Nr. 12, 7. Juni 1989, Seiten 4324-4328, American Chemical Society, Gaston, PA., US; L.A. DELEARIE et al.: "Technetium complexes of 3,5-Di-tert-butylcatechol. Direct synthesis of Tris(3,5-di-tert-butylcatecholato)technetium(VI) and Bis(3,5-di-tert-butylcatecholato)-(di-tert-butylamidophenolato)technetium(VI) from ammonium pertechnetate" * Seite 4328: "Discussion" * --- | 1 | |
| D,X | ANGEWANDTE CHEMIE, Band 98, Nr. 2, 1986, Seiten 173-174, VCH Verlagsgesellschaft mbH, Weinheim, DE; B. WOLFF et al.: "Neuartige oktaedrische Si- und Ge-Komplexe mit einem sechszähnigen Diphenolliganden" --- | 1-3,5,7,10 | RECHERCHIERTE SACHGEBIETE (Int.Cl.5) |
| A | * Seite 173; Beispiel 2B * --- | 1 | |
| X | * Seite 173; Beispiel 2A * --- | | |
| A | WO-A-9 000 854 (MALLINCKRODT) * Seite 42, Verbindung worin R5=OH; Anspruch 1 * --- | 1,8 | |
| A | EP-A-0 386 873 (AMERSHAM) * Seite 3; Anspruch 1 * --- | 1 | |
| P,A | EP-A-0 417 870 (INSTITUT FR DIAGNOSTIKFORSCHUNG) * Anspruch 1 * --- | 1,8 | |
| A | EP-A-0 163 119 (JOHNS HOPKINS UNIVERSITY) * Anspruch 1 * --- | 1,8 | |
| A | EP-A-0 251 494 (BOARD OF TRUSTEES LELAND STANFORD JUNIOR UNIVERSITY) * Seite 7, Zeilen 16-24; Anspruch 1 * ----- | 1,6,8 | |

EPO FORM 1503 03.82 (P0412)